(19) Europäisches Patentamt
European Patent Office
Office européen des brevets

(11) EP 4 322 236 A1

(12) EUROPEAN PATENT APPLICATION
published in accordance with Art. 153(4) EPC

(43) Date of publication:
**14.02.2024 Bulletin 2024/07**

(21) Application number: **22784564.1**

(22) Date of filing: **28.03.2022**

(51) International Patent Classification (IPC):
***H01L 51/50*** (2006.01) ***C07D 487/16*** (2006.01)
***C09K 11/06*** (2006.01)

(52) Cooperative Patent Classification (CPC):
**C07D 487/16; C09K 11/06; H10K 50/00**

(86) International application number:
**PCT/JP2022/015058**

(87) International publication number:
**WO 2022/215580 (13.10.2022 Gazette 2022/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.04.2021 JP 2021066013**

(71) Applicant: **NIPPON STEEL Chemical & Material Co., Ltd.**
**Tokyo 103-0027 (JP)**

(72) Inventors:
- **INOUE, Munetomo**
  **Tokyo 103-0027 (JP)**
- **KITERA, Sayuri**
  **Tokyo 103-0027 (JP)**
- **MATSUZAKI, Yoichi**
  **Tokyo 100-8071 (JP)**
- **SEINO, Moto**
  **Tokyo 100-8071 (JP)**
- **JONO, Maho**
  **Tokyo 100-8071 (JP)**

(74) Representative: **Kraus & Lederer PartGmbB**
**Thomas-Wimmer-Ring 15**
**80539 München (DE)**

# (54) LIGHT-EMITTING MATERIAL, AND ORGANIC ELECTROLUMINESCENT ELEMENT

(57) Provided are an emission material and an organic EL device including the emission material and having high emission efficiency and a long lifetime. An emission material represented by the following general formula (1) or the like, and an organic EL device including the emission material in a light emitting layer: wherein $A^1$ represents $CR^1$, C or N, each ring E represents a heterocycle represented by formula (1a), $X^1$ represents a group represented by, for example, O, S, Se, or N-$L^1$-$(Ar^1)_e$; and a, b, c, and d represent 0 or 1, provided that, when $R^1$ or $L^1$ does not form a fused ring with an adjacent aromatic ring, a + b + c + d = 1 is not satisfied.

EP 4 322 236 A1

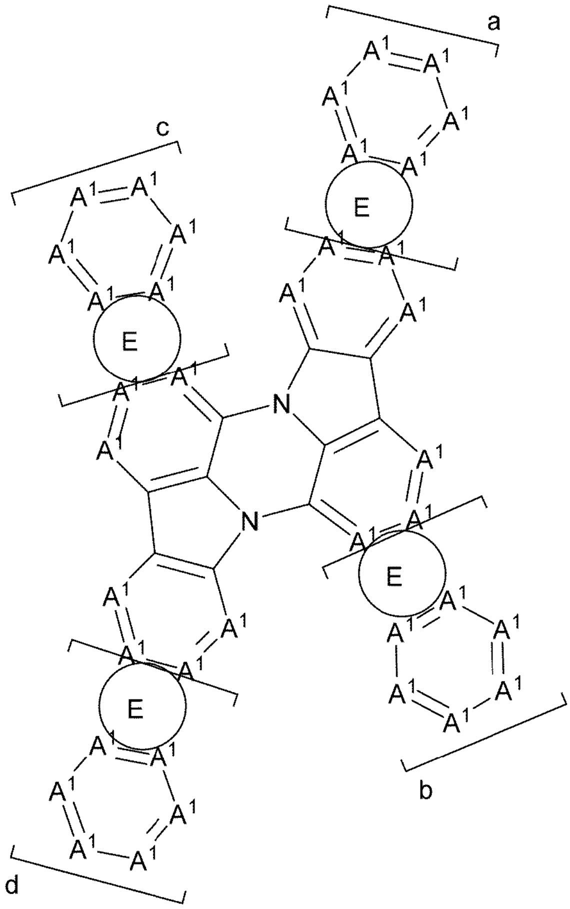

(1)

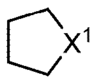

(1a)

[FIG. 1]

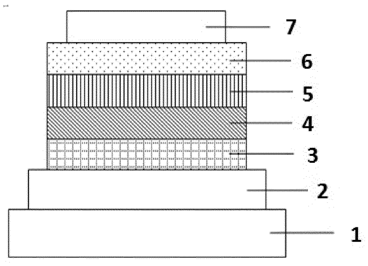

## Description

### Technical Field

The present invention relates to a light-emitting material and an organic electroluminescent device (also referred to as an organic EL device) including the light-emitting material as a light emitting layer.

When a voltage is applied to an organic EL device, holes and electrons are injected from the anode and the cathode, respectively, into the light emitting layer. Then, the injected holes and electrons are recombined in the light emitting layer to thereby generate excitons. At this time, according to the electron spin statistics theory, singlet excitons and triplet excitons are generated at a ratio of 1:3. In the fluorescent organic EL device that uses emission caused by singlet excitons, the limit of the internal quantum efficiency is said to be 25%. On the other hand, it has been known that, in the phosphorescent organic EL device that uses emission caused by triplet excitons, the internal quantum efficiency can be enhanced up to 100% when intersystem crossing efficiently occurs from singlet excitons.

A technology for extending the lifetime of a phosphorescent organic EL device has advanced in recent years, and the device is being applied to a display of a mobile phone and others. Regarding a blue organic EL device, however, a practical phosphorescent organic EL device has not been developed, and thus the development of a blue organic EL device having high efficiency and a long lifetime is desired.

Further, a highly efficient delayed fluorescence organic EL device utilizing delayed fluorescence has been developed, in recent years. For example, Patent Literature 1 discloses an organic EL device utilizing the Triplet-Triplet Fusion (TTF) mechanism, which is one of the mechanisms of delayed fluorescence. The TTF mechanism utilizes a phenomenon in which a singlet exciton is generated by the collision of two triplet excitons, and it is believed that the internal quantum efficiency can be enhanced up to 40%, in theory. However, its efficiency is low as compared with the efficiency of the phosphorescent organic EL device, and thus further improvement in efficiency is desired.

On the other hand, Patent Literature 2 discloses an organic EL device utilizing the Thermally Activated Delayed Fluorescence (TADF) mechanism. The TADF mechanism utilizes a phenomenon in which reverse intersystem crossing occurs from the triplet exciton to the singlet exciton in a material having a small energy difference between the singlet level and the triplet level, and it is believed that the internal quantum efficiency can be enhanced up to 100%, in theory. Specifically, Patent Literature 2 discloses an indolocarbazole compound as a thermally activated delayed fluorescence material.

Patent Literature 3, Patent Literature 4, and Patent Literature 5 each disclose a material including a polycyclic aromatic compound containing an indolophenazine backbone, and an organic EL device including the material. However, there is not disclosed any organic EL device including a material including a polycyclic aromatic compound containing an indolophenazine backbone, as an emission material.

Non Patent Literature 1 discloses a material including a polycyclic aromatic compound containing a carbazole backbone as a partial backbone of an indolophenazine backbone, and a blue organic EL device including the material as an emission material. However, these have no emission efficiency that can withstand practical use.

### Citation List

#### Patent Literature

Patent Literature 1: WO2010/134350
Patent Literature 2: WO2011/070963
Patent Literature 3: JP2012-234873 A
Patent Literature 4: WO2014/008967
Patent Literature 5: CN111892607

#### Non Patent Literature

Non Patent Literature 1: Journal of Material Chemistry C, 2017, Volume 5, Issue 3, 709

### Summary of Invention

#### Technical Problem

In view of applying an organic EL device to a display device such as a flat panel display and a light source, it

is necessary to improve the emission efficiency of the device and sufficiently ensure the stability of the device at the time of driving, at the same time. The present invention has been made under such circumstances, and an object thereof is to provide an emission material that can be used to obtain a practically useful organic EL device having high emission efficiency and high driving stability, and an organic EL device including the emission material.

Solution to Problems

Specifically, the present invention is an emission material represented by the following general formula (1) or (1-1).

[C1]

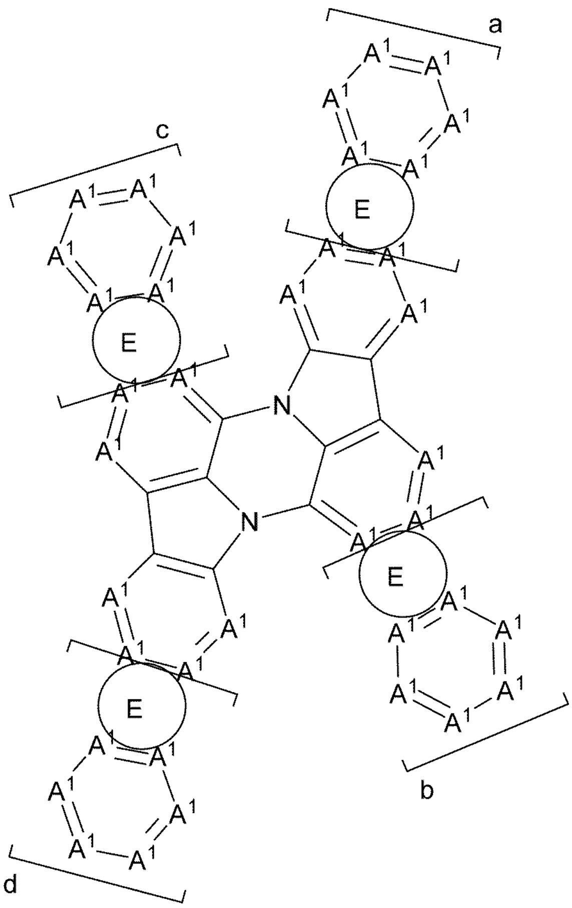

(1)

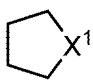

(1a)

In the formula, each $A^1$ independently represents $CR^1$, C or N, provided that the number of N present in one 6-membered ring containing $A^1$ in the general formula (1) is 2 or less. Each $R^1$ independently represents hydrogen, a cyano group, deuterium, a substituted or unsubstituted diarylamino group having 12 to 44 carbon atoms, a substituted or unsubstituted arylheteroarylamino group having 12 to 44 carbon atoms, a substituted or unsubstituted diheteroarylamino group having 12 to 44 carbon atoms, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups. When $R^1$ represents a substituted or unsubstituted diarylamino group, arylheteroarylamino group, diheteroarylamino group or aromatic hydrocarbon group, or a substituted or unsubstituted aromatic heterocyclic group, $R^1$ optionally forms one or more bonds with an aromatic ring having $A^1$ to which $R^1$ is bonded in the formula (1),

directly or via -O-, -S-, $-Si(R^a)_2-$, $-C(R^a)_2-$, or $-NR^a-$, to form a fused ring. Each $R^a$ independently represents hydrogen, deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups.

Each ring E independently represents a heterocycle represented by formula (1a), and each ring E is fused with an adjacent ring at any position. Each $X^1$ independently represents a group represented by $Si(R^d)_2$, $C(R^d)_2$, O, S, Se, or $N-L^1-(Ar^1)_e$. Each $R^d$ independently represents hydrogen, deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups. Each $L^1$ independently represents a substituted or unsubstituted aromatic hydrocarbon group having 6 to 30 carbon atoms, or a substituted or unsubstituted aromatic heterocyclic group having 3 to 30 carbon atoms, and each $Ar^1$ independently represents a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 these aromatic rings. $L^1$ optionally forms one bond with an aromatic ring adjacent to a heterocycle to which $L^1$ is bonded, directly or via -O-, -S-, $-Si(R^c)_2-$, $- C(R^c)_2-$, or $-NR^c-$, to form a fused ring. Each $R^c$ independently represents hydrogen, deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups. a, b, c, and d each independently represent 0 or 1, and each e independently represents an integer of 0 to 5, provided that, when $R^1$ or $L^1$ does not form a fused ring with an aromatic ring to which $R^1$ or $L^1$ is bonded or with an aromatic ring adjacent thereto, a + b + c + d = 1 is not satisfied.

At least one $R^1$ or $L^1$ may form one or more bonds with an aromatic having $A^1$ to which $R^1$ is bonded or with an aromatic ring adjacent to $L^1$ in the formula (1), directly or via -O-, -S-, $-Si(R^a)_2-$, $-C(R^a)_2-$, or $-NR^a-$, to form a fused ring. At least one $L^1$ may be directly bonded to an adjacent aromatic ring to form a fused ring. At least one $R^1$ may be a substituted or unsubstituted carbazolyl group, and may form one or more bonds with an aromatic ring to which the carbazolyl group is bonded, directly or via -O-, -S-, $-Si(R^a)_2-$, $-C(R^a)_2-$, or $-NR^a-$, to form a fused ring.

[C2]

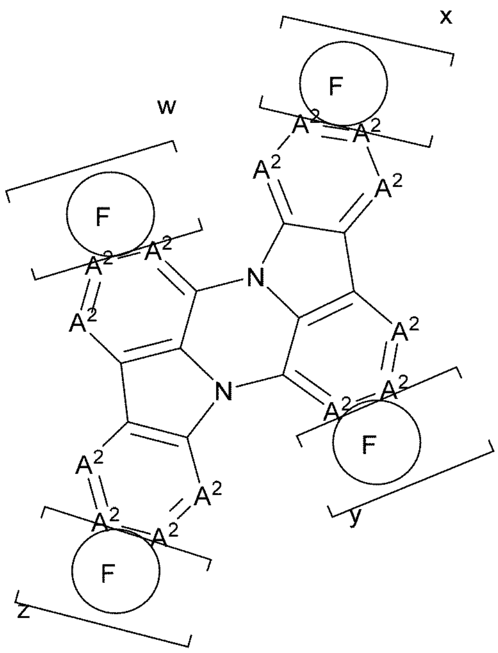

(1－1)

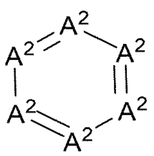

(1－1a)

Each $A^2$ independently represents $CR^2$, C or N, provided that the number of N present in one 6-membered ring containing $A^2$ in the general formula (1-1) is 2 or less. Each $R^2$ independently represents hydrogen, a cyano group, deuterium, a substituted or unsubstituted diarylamino group having 12 to 44 carbon atoms, a substituted or unsubstituted arylheteroarylamino group having 12 to 44 carbon atoms, a substituted or unsubstituted diheteroarylamino group having 12 to 44 carbon atoms, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups. When $R^2$ represents a substituted or unsubstituted diarylamino group, arylheteroarylamino group, diheteroarylamino group or aromatic hydrocarbon group, or a substituted or unsubstituted aromatic heterocyclic group, $R^2$ optionally forms one or more bonds with an aromatic ring having $A^2$ to which $R^2$ is bonded in the formula (1-1), directly or via -O-, -S-, $-Si(R^b)_2-$, - $C(R^b)_2-$, or $-NR^b-$, to form a fused ring. Each $R^b$ independently represents hydrogen, deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups. Each ring F independently represents an aromatic ring represented by formula (1-1a), and each ring F is fused with an adjacent ring at any position.

x, y, w, and z each independently represent 0 or 1.

In the general formula (1), $a + b + c + d \geq 2$ may be satisfied. In addition, b and c may be each 1, or a and d may be each 1.

In the general formula (1-1), $x + y + z + w \geq 2$ may be satisfied. In addition, x and z may be each 1, or y and w may be each 1.

The emission material represented by the general formula (1) can be an emission material represented by the following general formula (2) or general formula (3). Herein, a ring E has the same meaning as in the general formula (1).

[C3]

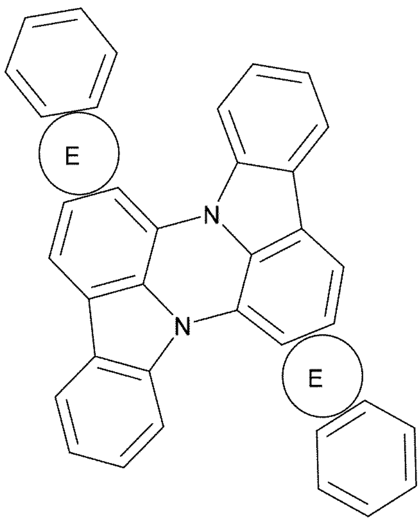

(2)

[C4]

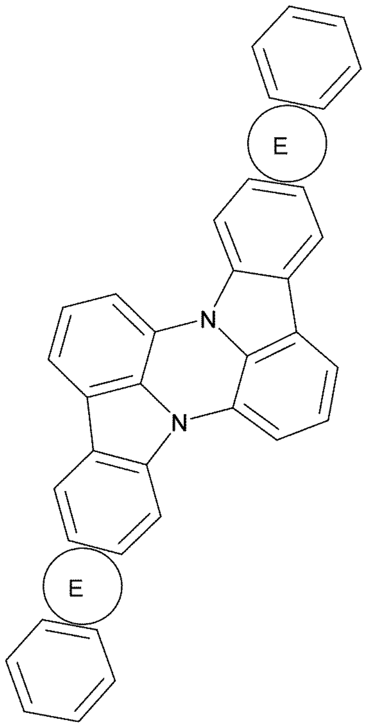

(3)

Each ring E described above may independently represent a group in which $X^1$ in the formula (1a) is represented by $N-L^1-(Ar^1)_e$.

The emission material represented by the general formula (1) or general formula (1-1) can be an emission material represented by the following general formula (4) .

[C5]

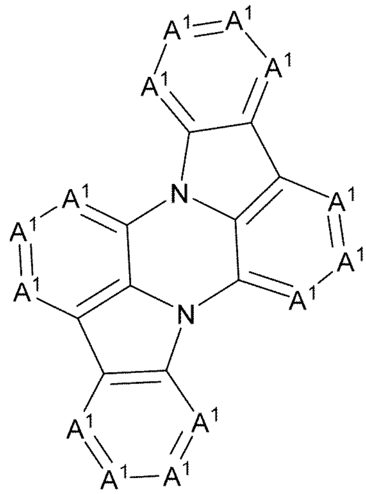

(4)

In the formula, each $A^1$ independently represents $CR^1$ or N. $R^1$ has the same meaning as $R^1$ in the general formula (1), provided that the number of N present in one 6-membered ring containing $A^1$ in the general formula (4) is 2 or less.

In the emission material represented by the general formula (1) or general formula (1-1), a difference (ΔEST) between a singlet excited energy (S1) and a triplet excited energy (T1) is preferably 0.40 eV or less.

The present invention is also an organic electroluminescent device comprising one or more light emitting layers between an anode and a cathode opposite to each other, wherein at least one of the light emitting layers contains the emission material.

Advantageous Effect of Invention

According to the emission material of the present invention, a practically useful organic EL device having high emission efficiency and high driving stability can be obtained. The emission material of the present invention exhibits the maximum wavelength in a blue, cyan, or green spectral region. The emission material exhibits the maximum wavelength particularly at 410 nm to 550 nm, preferably 430 nm to 495 nm. The photoluminescence quantum yield of the emission material of the present invention can reach 40% or more. The emission material of the present invention is used to thereby provide a higher-efficiency device. An organic EL device having a light emitting layer including the emission material has a high emission efficiency, and a color.

Brief Description of Drawing

[Figure 1] Figure 1 shows a schematic cross-sectional view of a structure example of the organic EL device used in the present invention.

Description of Embodiments

The emission material of the present invention is represented by the general formula (1) or general formula (1-1). The emission material is preferably an emission material represented by the general formula (2), general formula (3), or general formula (4). The organic EL device of the present invention has one or more light emitting layers between an anode and a cathode opposite to each other, and at least one of the light emitting layers contains the compound represented by the general formula (1) or general formula (1-1), as an emission material. The organic EL device has a plurality of layers between an anode and a cathode opposite to each other, at least one layer of the plurality of layers is a light emitting layer, and the light emitting layer may contain a host material, as necessary. The general formula (1) will be described below. The compound represented by the general formula (1) typically has a structure in which a plurality of indole rings is fused with a phenazine ring, or a structure similar thereto.

In the general formula (1), $A^1$ represents $CR^1$, N, or a carbon atom, provided that the number of N in $A^1$ present in one 6-membered ring containing $A^1$ in the general formula (1) is 2 or less. The 6-membered ring containing $A^1$ may be fused with an adjacent ring E, and in this case, two of $A^1$ are carbon atoms and such carbon atoms are shared with the ring E.

Each $R^1$ independently represents hydrogen, a cyano group, deuterium, a substituted or unsubstituted diarylamino group having 12 to 44 carbon atoms, a substituted or unsubstituted arylheteroarylamino group having 12 to 44 carbon atoms, a substituted or unsubstituted diheteroarylamino group having 12 to 44 carbon atoms, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups. Each $R^1$ preferably represents hydrogen, a cyano group, deuterium, a substituted or unsubstituted diarylamino group having 12 to 24 carbon atoms, a substituted or unsubstituted arylheteroarylamino group having 12 to 24 carbon atoms, a substituted or unsubstituted diheteroarylamino group having 12 to 24 carbon atoms, an aliphatic hydrocarbon group having 1 to 8 carbon atoms, a substituted or unsubstituted aromatic hydrocarbon group having 6 to 20 carbon atoms, a substituted or unsubstituted aromatic heterocyclic group having 3 to 20 carbon atoms, or a substituted or unsubstituted linked aromatic group formed by linking 2 to 6 such aromatic groups. Each $R^1$ more preferably represents hydrogen, a substituted or unsubstituted diarylamino group having 12 to 18 carbon atoms, a substituted or unsubstituted arylheteroarylamino group having 12 to 18 carbon atoms, an aliphatic hydrocarbon group having 1 to 4 carbon atoms, a substituted or unsubstituted aromatic hydrocarbon group having 6 to 10 carbon atoms, a substituted or unsubstituted aromatic heterocyclic group having 3 to 12 carbon atoms, or a substituted or unsubstituted linked aromatic group formed by linking 2 to 4 such aromatic groups.

When $R^1$ represents the unsubstituted diarylamino group, the unsubstituted arylheteroarylamino group, the unsubstituted diheteroarylamino group, or the aliphatic hydrocarbon group, specific examples of $R^1$ include diphenylamino, dibiphenylamino, phenylbiphenylamino, naphthylphenylamino, dinaphthylamino, dianthranilamino, diphenanthrenylamino, dibenzofuranylphenylamino, dibenzofuranylbiphenylamino, bisdibenzofuranylamino, methyl, ethyl, propyl, butyl, pentyl, hexyl, heptyl, octyl, nonyl, and decyl. Preferred examples thereof include diphenylamino, dibiphenylamino, phenylbiphenylamino, naphthylphenylamino, dinaphthylamino, dibenzofuranylphenylamino, dibenzofuranylbiphenylamino, bisdibenzofuranylamino, methyl, ethyl, propyl, butyl, pentyl, hexyl, heptyl, and octyl. More preferred examples thereof include diphenylamino, phenylbiphenylamino, dibenzofuranylphenylamino, dibenzofuranylbiphenylamino, and butyl. When $R^1$ represents the aliphatic hydrocarbon group, $R^1$ may be linear, branched, or cyclic.

When $R^1$ represents the unsubstituted aromatic hydrocarbon group, aromatic heterocyclic group, or linked aromatic group, specific examples thereof include a group produced by removing one hydrogen atom from benzene, naphthalene, acenaphthene, acenaphthylene, azulene, anthracene, chrysene, pyrene, phenanthrene, triphenylene, fluorene, benzo[a]anthracene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzisothiazole, benzothiadiazole, purine, pyranone, coumarin, isocoumarin, chromone, dibenzofuran, dibenzothiophene, dibenzoselenophene, carbazole, indolocarbazole, and a compound formed by linking 2 to 9 of these compounds. Preferred examples thereof include a group produced by removing one hydrogen atom from benzene, naphthalene, acenaphthene, acenaphthylene, azulene, anthracene, chrysene, pyrene, phenanthrene, fluorene, benzo[a] anthracene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzisothiazole, benzothiadiazole, purine, pyranone, coumarin, isocoumarin, chromone, dibenzofuran, dibenzothiophene, dibenzoselenophene, carbazole, and a compound formed by linking 2 to 6 of these compounds. More preferred examples thereof include a group produced by removing one hydrogen atom from benzene, naphthalene, azulene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzoisothiazole, benzothiadiazole, purine, pyranone, coumarin, isocoumarin, chromone, dibenzofuran, dibenzothiophene, dibenzoselenophene, carbazole, and a compound formed by linking 2 to 4 of these compounds.

Herein, each of the aromatic hydrocarbon groups, the aromatic heterocyclic groups and the linked aromatic groups may have a substituent. The same also applies to the aryl groups and heteroaryl groups contained in the diarylamino group, the arylheteroarylamino group, and the diheteroarylamino group.

When these groups have a substituent, the substituent is a cyano group, an aliphatic hydrocarbon group having 1 to 10 carbon atoms, a diarylamino group having 12 to 30 carbon atoms, an arylheteroarylamino group having 12 to 30 carbon atoms, a diheteroarylamino group having 12 to 30 carbon atoms, an alkoxy group having 1 to 10 carbon atoms, an aryloxy group having 6 to 18 carbon atoms, an alkylthio group having 1 to 10 carbon atoms, or an arylthio group having 6 to 18 carbon atoms. When the substituent is an aliphatic hydrocarbon group having 1 to 10 carbon atoms, the substituent may be linear, branched, or cyclic. When the diarylamino group, the arylheteroarylamino group, the diheteroarylamino group, the aryloxy group, or the arylthio group substitutes the aryl group or the heteroaryl group contained in the aromatic hydrocarbon group, the aromatic heterocyclic group, the aromatic ring of the linked aromatic group, or the diarylamino group, the arylheteroarylamino group or the diheteroarylamino group, nitrogen and carbon, oxygen and carbon, or sulfur and carbon are bound by a single bond. The number of substituents is 0 to 5, and preferably 0 to 2. When each of the aromatic hydrocarbon groups and aromatic heterocyclic groups has a substituent, the number of carbon atoms of the substituent is not included in the calculation of the number of carbon atoms. However, it is preferred that the total number of carbon atoms including the number of carbon atoms of the substituent satisfy the above range.

Specific examples of the substituent include cyano, methyl, ethyl, propyl, butyl, pentyl, hexyl, heptyl, octyl, nonyl, decyl, diphenylamino, naphthylphenylamino, dinaphthylamino, dianthranilamino, diphenanthrenylamino, dibenzofuranylphenylamino, dibenzofuranylbiphenylamino, bisdibenzofuranylamino, methoxy, ethoxy, phenol, diphenyloxy, methylthio, ethylthio, thiophenol, and diphenylthio. Preferred examples thereof include cyano, methyl, ethyl, propyl, butyl, pentyl, hexyl, heptyl, octyl, diphenylamino, naphthylphenylamino, dinaphthylamino, dibenzofuranylphenylamino, dibenzofuranylbiphenylamino, bisdibenzofuranylamino, phenol, and thiophenol.

Herein, the linked aromatic group refers to an aromatic group in which carbon atoms in the aromatic ring of the aromatic group are linked to each other. It refers to an aromatic group in which two or more aromatic groups are linked, and these may be linear or branched. Such aromatic groups may be each an aromatic hydrocarbon group or an aromatic heterocyclic group, and such a plurality of aromatic groups may be the same or different. The aromatic group corresponding to the linked aromatic group is different from a substituted aromatic group.

It is herein understood that hydrogen may also be deuterium. In other words, some or all of H atoms contained in backbones such as carbazole and substituents such as $R^1$ and $Ar^1$ in the general formulas (1) to (4), (1-1) and the like may be deuterium.

Each ring E independently represents a heterocycle represented by formula (1a), and each ring E is fused with an adjacent ring at any position.

Each $X^1$ independently represents a divalent group represented by $Si(R^d)_2$, $C(R^d)_2$, O, S, Se, or $N\text{-}L^1\text{-}(Ar^1)_e$, preferably O, S, or $N\text{-}L^1\text{-}(Ar^1)_e$, more preferably $N\text{-}L^1\text{-}(Ar^1)_e$.

Each $R^d$ independently represents hydrogen, deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups. Each $R^d$ represents hydrogen, deuterium, an aliphatic hydrocarbon group having 1 to 8 carbon atoms, a substituted or unsubstituted aromatic hydrocarbon group having 6 to 20 carbon atoms,

a substituted or unsubstituted aromatic heterocyclic group having 3 to 20 carbon atoms, or a substituted or unsubstituted linked aromatic group formed by linking 2 to 6 such aromatic groups. Each $R^d$ more preferably represents hydrogen, an aliphatic hydrocarbon group having 1 to 4 carbon atoms, a substituted or unsubstituted aromatic hydrocarbon group having 6 to 10 carbon atoms, a substituted or unsubstituted aromatic heterocyclic group having 2 to 12 carbon atoms, or a substituted or unsubstituted linked aromatic group formed by linking 2 to 4 such aromatic groups.

When $R^d$ represents an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups, specific examples of $R^d$ are the same as in the case of $R^1$.

Each $L^1$ independently represents a substituted or unsubstituted aromatic hydrocarbon group having 6 to 30 carbon atoms or a substituted or unsubstituted aromatic heterocyclic group having 3 to 30 carbon atoms. Each $L^1$ preferably represents a substituted or unsubstituted aromatic hydrocarbon group having 6 to 20 carbon atoms or a substituted or unsubstituted aromatic heterocyclic group having 2 to 20 carbon atoms. Each $L^1$ more preferably represents a substituted or unsubstituted aromatic hydrocarbon group having 6 to 10 carbon atoms or a substituted or unsubstituted aromatic heterocyclic group having 2 to 12 carbon atoms.

Specific examples of the unsubstituted $L^1$ include a group produced by removing 1 + e hydrogen atoms from benzene, naphthalene, acenaphthene, acenaphthylene, azulene, anthracene, chrysene, pyrene, phenanthrene, triphenylene, fluorene, benzo[a]anthracene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzisothiazole, benzothiadiazole, purine, pyranone, coumarin, isocoumarin, chromone, dibenzofuran, dibenzothiophene, dibenzoselenophene, and carbazole. Preferred examples thereof include a group produced by removing 1 + e hydrogen atoms from benzene, naphthalene, acenaphthene, acenaphthylene, azulene, anthracene, chrysene, pyrene, phenanthrene, fluorene, benzo[a]anthracene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzisothiazole, benzothiadiazole, purine, pyranone, coumarin, isocoumarin, chromone, dibenzofuran, dibenzothiophene, dibenzoselenophene, and carbazole. More preferred examples thereof include a group produced by removing 1 + e hydrogen atoms from benzene, naphthalene, azulene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzisothiazole, benzothiadiazole, purine, pyranone, coumarin, isocoumarin, chromone, dibenzofuran, dibenzothiophene, dibenzoselenophene, and carbazole.

$Ar^1$ represents a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 aromatic rings of aromatic groups selected from the aromatic hydrocarbon group and the aromatic heterocyclic group. $Ar^1$ preferably represents a substituted or unsubstituted aromatic hydrocarbon group having 6 to 20 carbon atoms, a substituted or unsubstituted aromatic heterocyclic group having 3 to 20 carbon atoms, or a substituted or unsubstituted linked aromatic group formed by linking 2 to 6 aromatic rings of aromatic groups selected from the aromatic hydrocarbon group and the aromatic heterocyclic group. $Ar^1$ more preferably represents a substituted or unsubstituted aromatic hydrocarbon group having 6 to 10 carbon atoms, a substituted or unsubstituted aromatic heterocyclic group having 3 to 12 carbon atoms, or a substituted or unsubstituted linked aromatic group formed by linking 2 to 4 aromatic rings of aromatic groups selected from the aromatic hydrocarbon group and the aromatic heterocyclic group. The aromatic hydrocarbon group, the aromatic heterocyclic group and the linked aromatic group are also referred to as aromatic groups.

Specific examples of the unsubstituted aromatic groups include a group produced by removing one hydrogen atom from benzene, naphthalene, acenaphthene, acenaphthylene, azulene, anthracene, chrysene, pyrene, phenanthrene, triphenylene, fluorene, benzo[a]anthracene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzisothiazole, benzothiadiazole, purine, pyranone, coumarin, isocoumarin, chromone, dibenzofuran, dibenzothiophene, dibenzoselenophene, carbazole, and a compound formed by linking 2 to 9 of these compounds. Preferred examples thereof include a group produced by removing one hydrogen atom from benzene, naphthalene, acenaphthene, acenaphthylene, azulene, anthracene, chrysene, pyrene, phenanthrene, fluorene, benzo[a]anthracene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole,

benzisothiazole, benzothiadiazole, purine, pyranone, coumarin, isocoumarin, chromone, dibenzofuran, dibenzothiophene, dibenzoselenophene, carbazole, and a compound formed by linking 2 to 6 of these compounds. More preferred examples thereof include a group produced by removing one hydrogen atom from benzene, naphthalene, azulene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzisothiazole, benzothiadiazole, purine, pyranone, coumarin, isocoumarin, chromone, dibenzofuran, dibenzothiophene, dibenzoselenophene, carbazole, and a compound formed by linking 2 to 4 of these compounds.

a, b, c, and d each independently represent 0 or 1, and each e independently represents an integer of 0 to 5, provided that, when $R^1$ or $L^1$ does not form a fused ring with an adjacent aromatic ring, a + b + c + d = 1 is not satisfied. e is preferably 3 or less. Preferably, b and c are each 1, or a and d are each 1.

Preferred aspects of the general formula (1) are the general formula (2), general formula (3) and general formula (4). Each symbol common in the general formulas (1) to (4) has the same meaning. The general formula (2) corresponds to a structure in which both b and c are 1 and a and d are each 0 in the general formula (1). A ring E has the same meaning as in the general formula (1) .

The ring E in the general formula (2) is fused with an adjacent ring at any position, and in this case, can be any of the following formula (2-a) to formula (2-j) depending on the fusion position.

[C6]

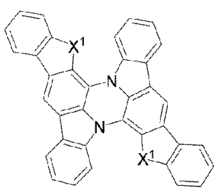
(2-a)

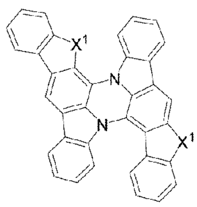
(2-b)

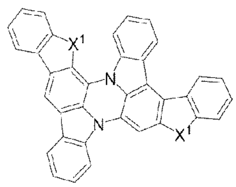
(2-c)

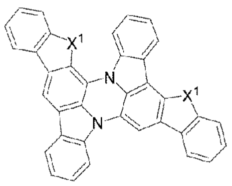
(2-d)

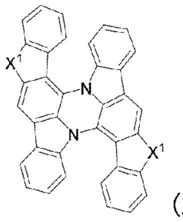
(2-e)

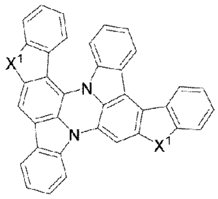
(2-f)

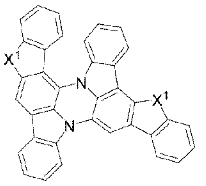
(2-g)

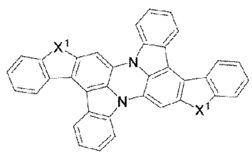
(2-h)

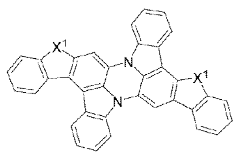
(2-i)

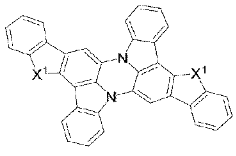
(2-j)

In the formulas, $X^1$ has the same meaning as in the general formula (1). Preferred is formula (2-a), (2-e), (2-h), or (2-j).

The general formula (3) corresponds to a structure in which both a and d are 1 and b and c are each 0 in the general formula (1). A ring E has the same meaning as in the general formula (1).

When the ring E is fused with an adjacent ring at any position in the general formula (3), any structure of the following formula (3-a) to formula (3-u) can be provided.

[C7]

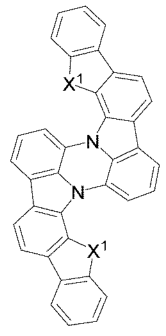
(3-a)
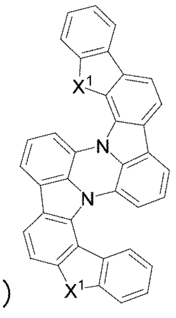
(3-b)
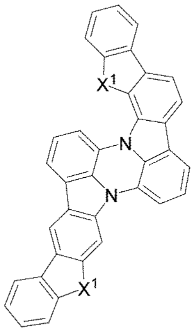
(3-c)

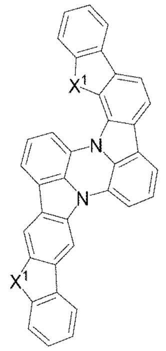
(3-d)
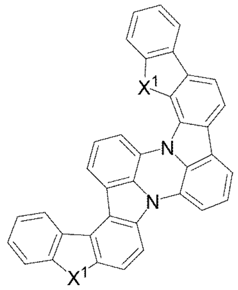
(3-e)
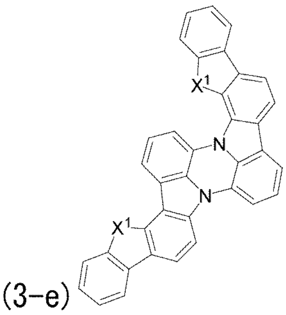
(3-f)

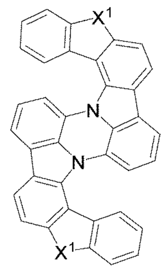
(3-g)
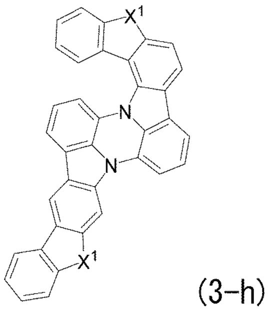
(3-h)
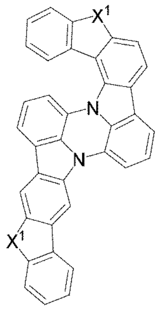
(3-i)

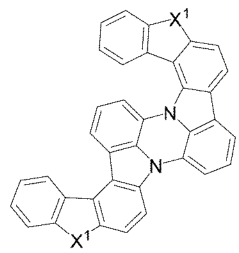
(3-j)
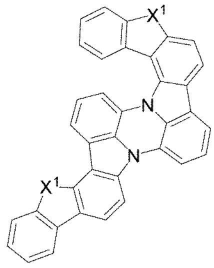
(3-k)
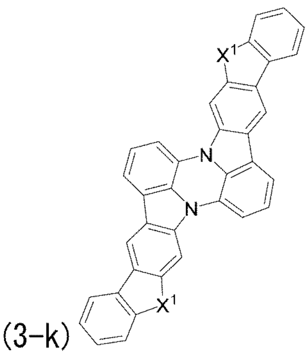
(3-l)

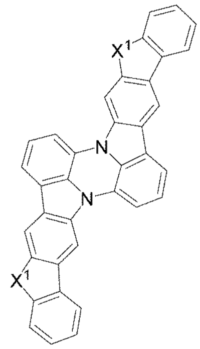
(3-m)
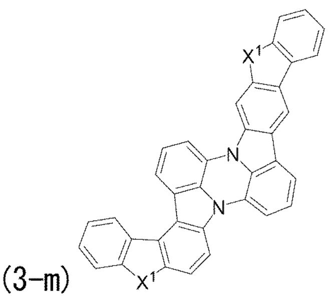
(3-n)
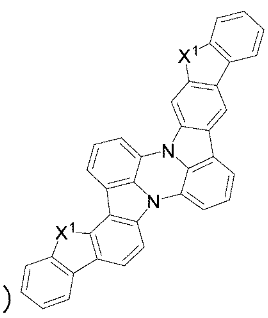
(3-o)

[C8]

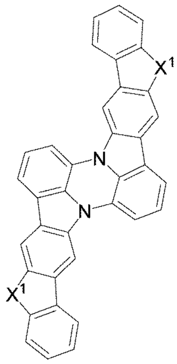
(3-p)

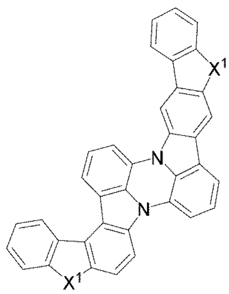
(3-q)

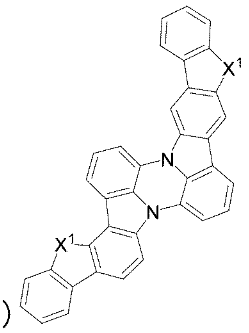
(3-r)

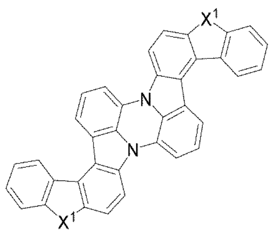
(3-s)

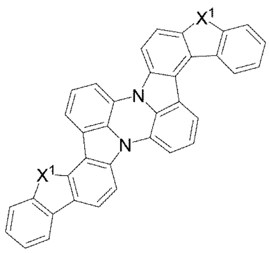
(3-t)

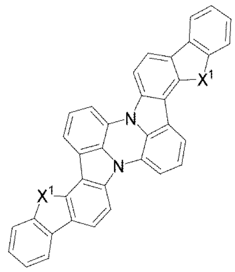
(3-u)

In the formulas, $X^1$ has the same meaning as in the general formula (1). Preferred are formula (3-a), (3-g), (3-1), (3-p), (3-s), and (3-u).

The general formula (4) corresponds to a structure in which all a, b, c and d are 0 in the general formula (1). In the general formula (4), each $A^1$ independently represents $CR^1$ or N. $R^1$ has the same meaning as $R^1$ in the general formula (1), provided that the number of N in $A^1$ present in one 6-membered ring containing $A^1$ in the general formula (4) is 2 or less.

When $R^1$ in the general formulas (1) to (4) represents a substituted or unsubstituted diarylamino group, arylheteroarylamino group, diheteroarylamino group or aromatic hydrocarbon group, or a substituted or unsubstituted aromatic heterocyclic group, $R^1$ optionally forms one or more bonds with an aromatic ring having $A^1$ to which $R^1$ is bonded in the formula (1), directly or via -O-, -S-, $-Si(R^a)_2-$, $-C(R^a)_2-$, or $-NR^a-$, to form a fused ring.

Each $R^a$ independently represents hydrogen, deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups. Each $R^a$ preferably represents hydrogen, deuterium, an aliphatic hydrocarbon group having 1 to 8 carbon atoms, a substituted or unsubstituted aromatic hydrocarbon group having 6 to 20 carbon atoms, a substituted or unsubstituted aromatic heterocyclic group having 3 to 20 carbon atoms, or a substituted or unsubstituted linked aromatic group formed by linking 2 to 6 such aromatic groups. Each $R^a$ more preferably represents hydrogen, an aliphatic hydrocarbon group having 1 to 4 carbon atoms, a substituted or unsubstituted aromatic hydrocarbon group having 6 to 10 carbon atoms, a substituted or unsubstituted aromatic heterocyclic group having 3 to 12 carbon atoms, or a substituted or unsubstituted linked aromatic group formed by linking 2 to 4 such aromatic groups.

When $R^a$ represents an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups, specific examples of $R^a$ are the same as in the case of $R^1$.

When $R^1$ in the general formulas (1) to (4) represents a substituted or unsubstituted diarylamino group, arylheteroarylamino group, diheteroarylamino group or aromatic hydrocarbon group, or a substituted or unsubstituted aromatic heterocyclic group and forms one bond with an aromatic ring to which $R^1$ is bonded, directly or via -O-, -S-, $-Si(R^a)_2-$, $-C(R^a)_2-$, or $-NR^a-$, to form a fused ring, any structure of the following exemplified compounds (D226) to (D236) can be provided.

When $R^1$ in the general formulas (1) to (4) represents a substituted or unsubstituted diarylamino group, arylheteroarylamino group, diheteroarylamino group or aromatic hydrocarbon group, or a substituted or unsubstituted aromatic heterocyclic group and forms two bonds with an aromatic ring to which $R^1$ is bonded, directly or via -O-, -S-, $-Si(R^a)_2-$, $-C(R^a)_2-$, or $-NR^a-$, to form a fused ring, any structure of the following exemplified compounds (D237) to (D240) can be provided.

$L^1$ in the general formulas (1) to (3) optionally form one or more bonds with an aromatic ring adjacent to a heterocycle to which $L^1$ is bonded, directly or via -O-, -S-, $-Si(R^c)_2-$, $-C(R^c)_2-$, or $-NR^c-$, to form a fused ring.

Each $R^c$ independently represents hydrogen, deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups. Each $R^c$ preferably represents hydrogen, deuterium, an aliphatic hydrocarbon group having 1 to 8 carbon atoms, a substituted or unsubstituted aromatic hydrocarbon group having 6 to 20 carbon atoms, a substituted or unsubstituted aromatic heterocyclic group having 3 to 20 carbon atoms, or a substituted or unsubstituted linked aromatic group formed by linking 2 to 6 such aromatic groups. Each $R^c$ more preferably represents hydrogen, an aliphatic hydrocarbon group having 1 to 4 carbon atoms, a substituted or unsubstituted aromatic hydrocarbon group having 6 to 10 carbon atoms, a substituted or unsubstituted aromatic heterocyclic group having 3 to 12 carbon atoms, or a substituted or unsubstituted linked aromatic group formed by linking 2 to 4 such aromatic groups.

When $R^c$ represents an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups, specific examples of $R^c$ are the same as in the case of $R^1$.

When $L^1$ in the general formulas (1) to (3) optionally forms one bond with an aromatic ring adjacent to a heterocycle to which $L^1$ is bonded, directly or via - O-, -S-, $-Si(R^c)_2-$, $-C(R^c)_2-$, or $-NR^c-$, to form a fused ring, any structure of the following exemplified compounds (D175) to (D224) and (D246) to (D254) can be provided.

The general formula (1-1) will be described below. The compound represented by the general formula (1-1) typically has a structure in which a plurality of benzene rings is fused with a phenazine ring, or a structure similar thereto. One preferred aspect is represented by the general formula (4).

In the general formula (1-1), $A^2$ represents $CR^2$, N, or a carbon atom, provided that the number of N in $A^2$ present in one 6-membered ring containing $A^2$ in the general formula (1-1) is 2 or less. The 6-membered ring containing $A^2$ may be fused with an adjacent ring F, and in this case, two of $A^2$ are carbon atoms and such carbon atoms are shared with the ring F.

Each $R^2$ independently represents hydrogen, a cyano group, deuterium, a substituted or unsubstituted diarylamino group having 12 to 44 carbon atoms, a substituted or unsubstituted arylheteroarylamino group having 12 to 44 carbon atoms, a substituted or unsubstituted diheteroarylamino group having 12 to 44 carbon atoms, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups. Each $R^2$ preferably represents hydrogen, a cyano group, deuterium, a substituted or unsubstituted diarylamino group having 12 to 24 carbon atoms, a substituted or unsubstituted arylheteroarylamino group having 12 to 24 carbon atoms, a substituted or unsubstituted diheteroarylamino group having 12 to 24 carbon atoms, an aliphatic hydrocarbon group having 1 to 8 carbon atoms, a substituted or unsubstituted aromatic hydrocarbon group having 6 to 20 carbon atoms, a substituted or unsubstituted aromatic heterocyclic group having 3 to 20 carbon atoms, or a substituted or unsubstituted linked aromatic group formed by linking 2 to 6 such aromatic groups. Each $R^2$ more preferably represents hydrogen, a substituted or unsubstituted diarylamino group having 12 to 18 carbon atoms, a substituted or unsubstituted arylheteroarylamino group having 12 to 18 carbon atoms, an aliphatic hydrocarbon group having 1 to 4 carbon atoms, a substituted or unsubstituted aromatic hydrocarbon group having 6 to 10 carbon atoms, a substituted or unsubstituted aromatic heterocyclic group having 3 to 12 carbon atoms, or a substituted or unsubstituted linked aromatic group formed by linking 2 to 4 such aromatic groups.

Specific examples of the unsubstituted $R^2$ are the same as in the case of $R^1$.

When $R^2$ in the general formula (1-1) represents a substituted or unsubstituted diarylamino group, arylheteroarylamino group, diheteroarylamino group or aromatic hydrocarbon group, or a substituted or unsubstituted aromatic heterocyclic group and $R^2$ forms one bond with an aromatic ring having $A^2$ to which $R^2$ is bonded in the formula (1-1), directly or via -O-, -S-, - $Si(R^b)_2-$, $-C(R^b)_2-$, or $-NR^b-$, to form a fused ring, any structure of the following exemplified compounds (D164), (D165), (D167), (D168), (D170), (D171), (D172), (D173), and (D174) can be provided.

When $R^2$ in the general formula (1-1) represents a substituted or unsubstituted diarylamino group, arylheteroarylamino group, diheteroarylamino group or aromatic hydrocarbon group, or a substituted or unsubstituted aromatic heterocyclic group and $R^2$ forms two bonds with an aromatic ring having $A^2$ to which $R^2$ is bonded in the formula (1-1), directly or via -O-, -S-, - $Si(R^b)_2-$, $-C(R^b)_2-$, or $-NR^b-$, to form a fused ring, any structure of the following exemplified compound (D169) can be provided.

Each $R^b$ independently represents hydrogen, deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups. Each $R^b$ preferably represents hydrogen, deuterium, an aliphatic hydrocarbon group having 1 to 8 carbon atoms, a substituted or unsubstituted aromatic hydrocarbon group having 6 to 20

carbon atoms, a substituted or unsubstituted aromatic heterocyclic group having 3 to 20 carbon atoms, or a substituted or unsubstituted linked aromatic group formed by linking 2 to 6 such aromatic groups. Each $R^b$ more preferably represents hydrogen, an aliphatic hydrocarbon group having 1 to 4 carbon atoms, a substituted or unsubstituted aromatic hydrocarbon group having 6 to 10 carbon atoms, a substituted or unsubstituted aromatic heterocyclic group having 2 to 12 carbon atoms, or a substituted or unsubstituted linked aromatic group formed by linking 2 to 4 such aromatic groups.

When $R^b$ represents an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups, specific examples of $R^b$ are the same as in the case of $R^1$.

Specific examples of the emission materials represented by the general formulas (1) to (4) and (1-1) are shown below, but the materials are not limited to these exemplified compounds.

[C9]

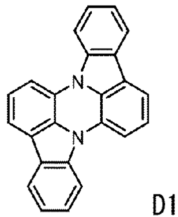
D1
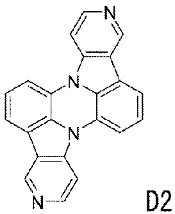
D2
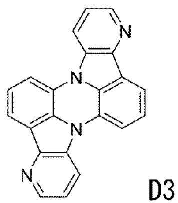
D3
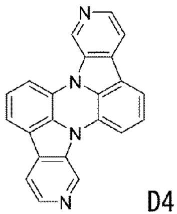
D4
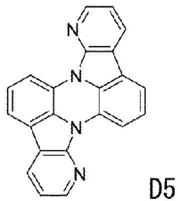
D5
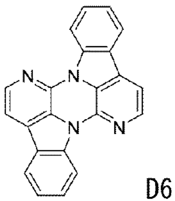
D6
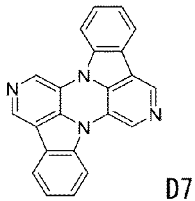
D7
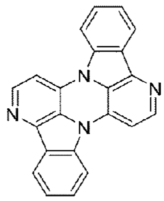
D8
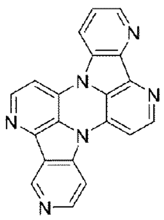
D9
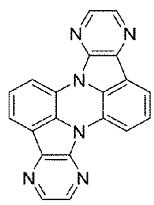
D10
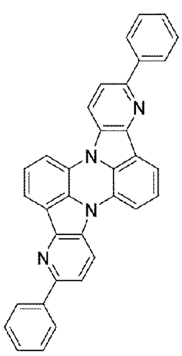
D11
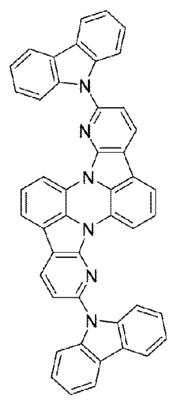
D12
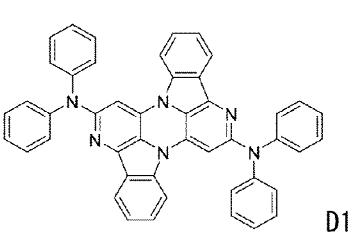
D13
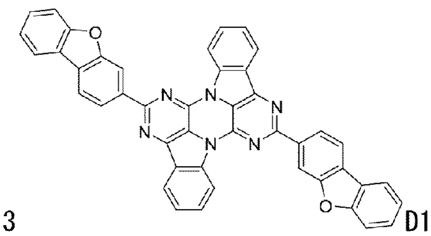
D14
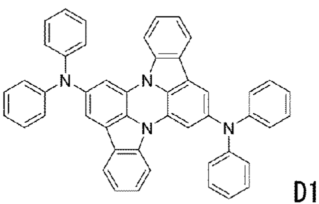
D15
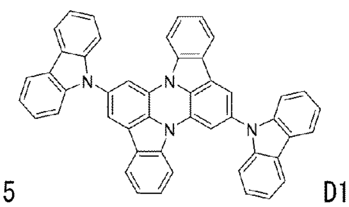
D16

[C10]

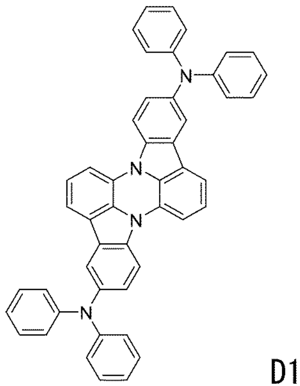
D17

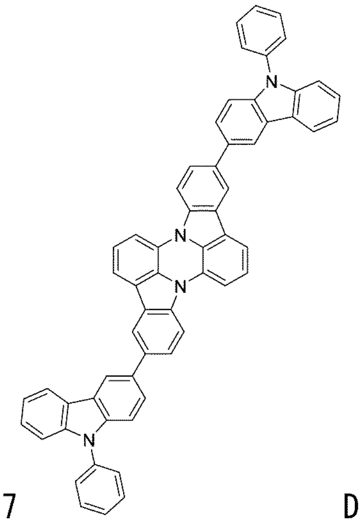
D18

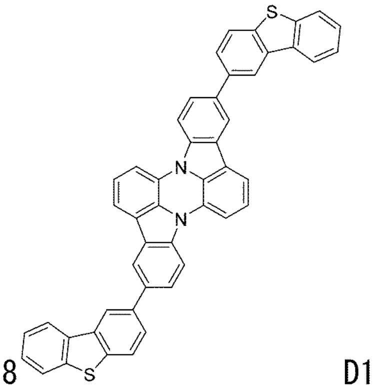
D19

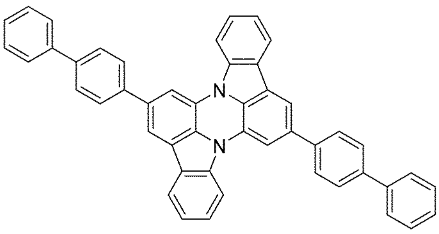
D20

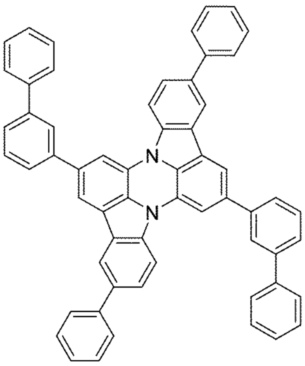
D21

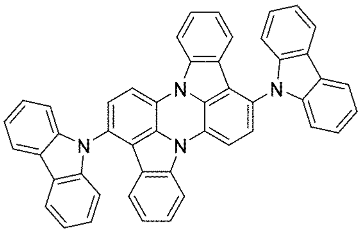
D22

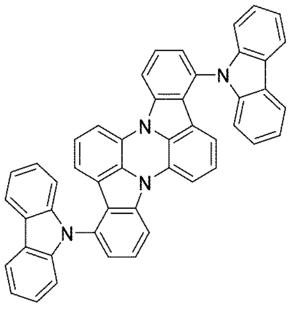
D23

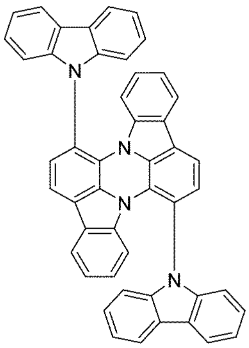
D24

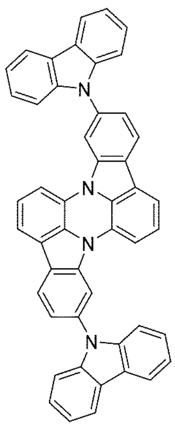
D25

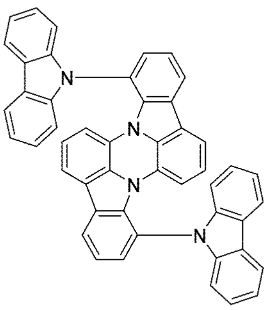
D26

[C11]

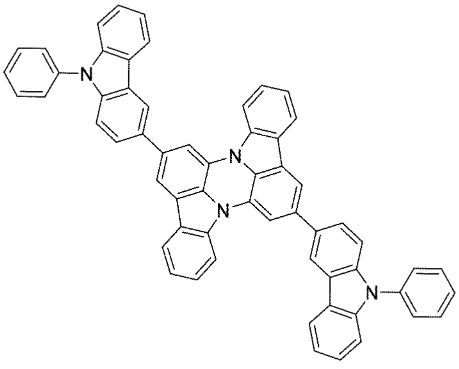
D27

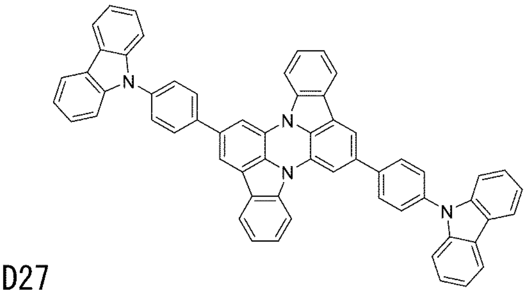
D28

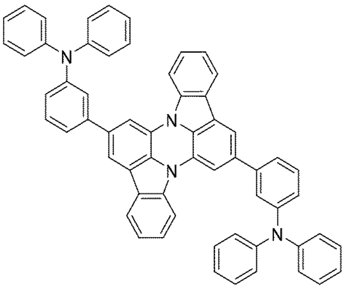
D29

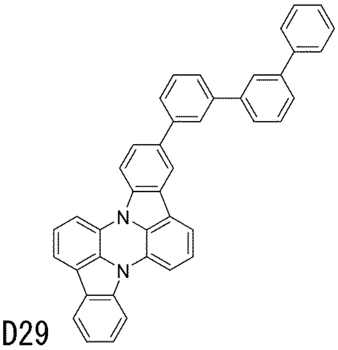
D30

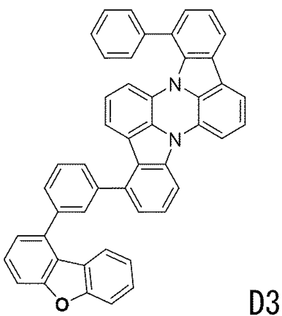
D31

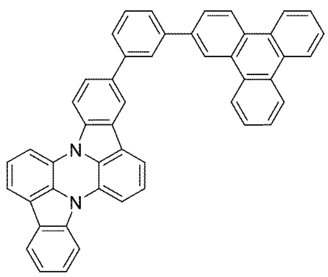
D32

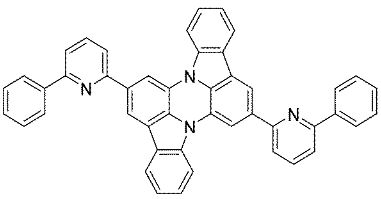
D33

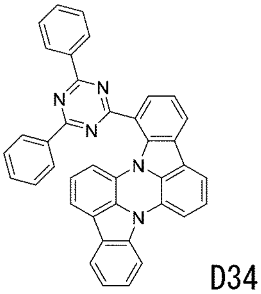
D34

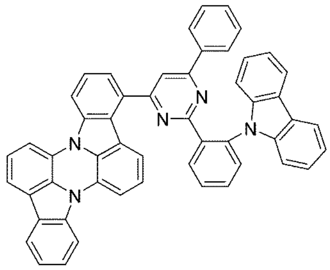
D35

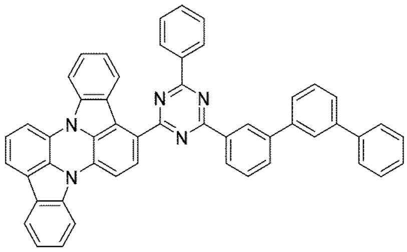
D36

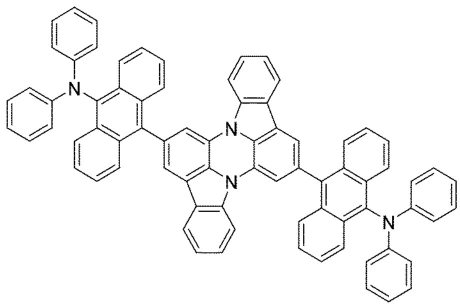
D37

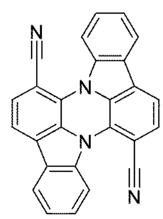
D38

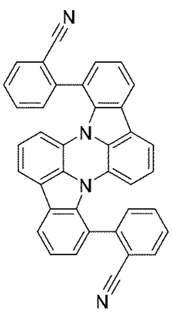
D39

[C12]

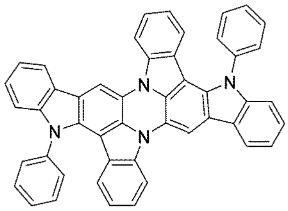
D40
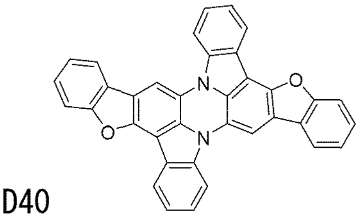
D41
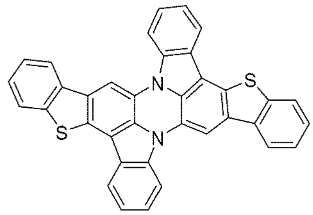
D42
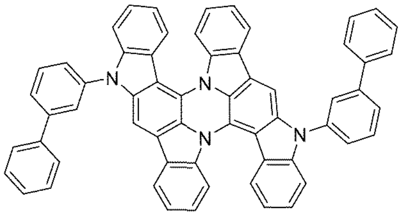
D43
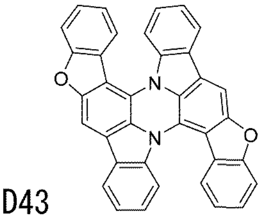
D44
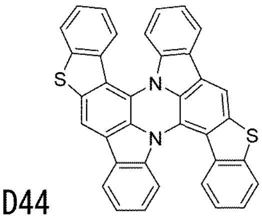
D45
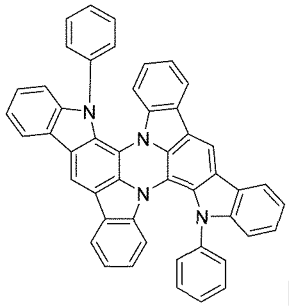
D46
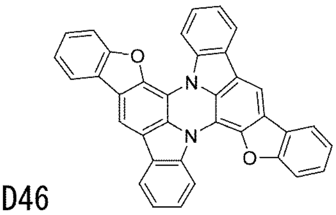
D47
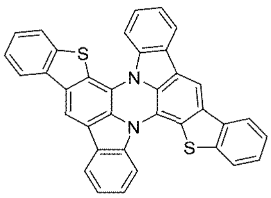
D48
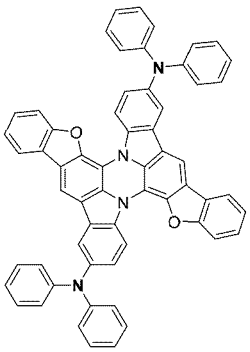
D49
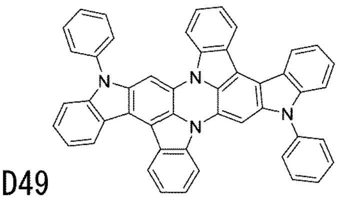
D50
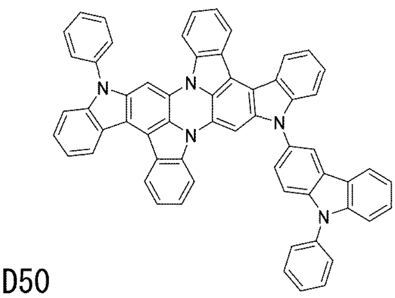
D51
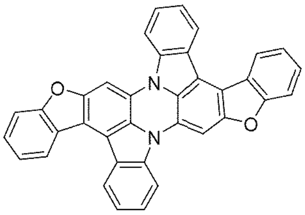
D52
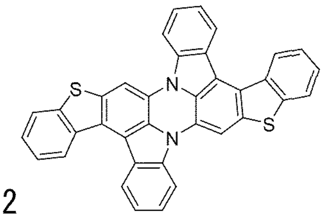
D53
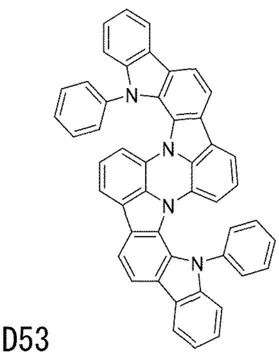
D54

[C13]

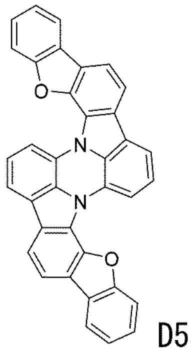
D55

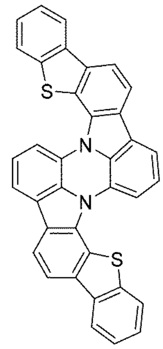
D56

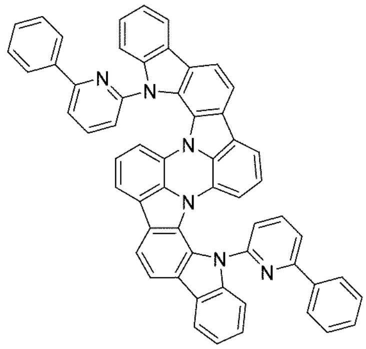
D57

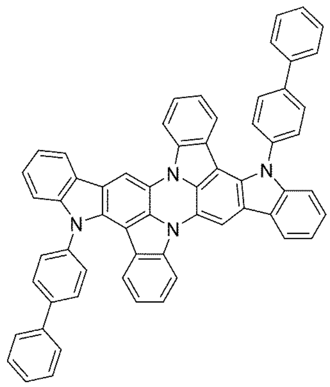
D58

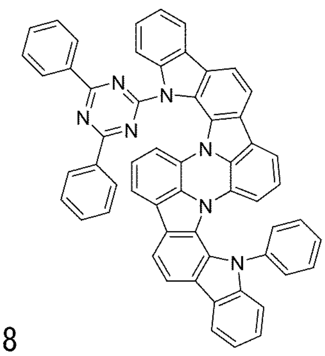
D59

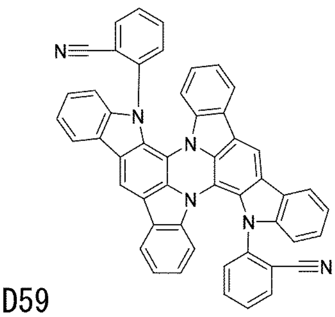
D60

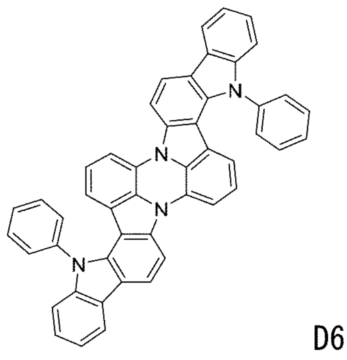
D61

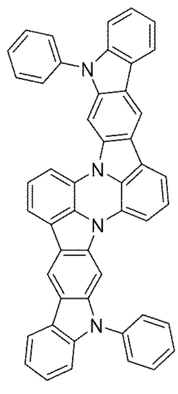
D62

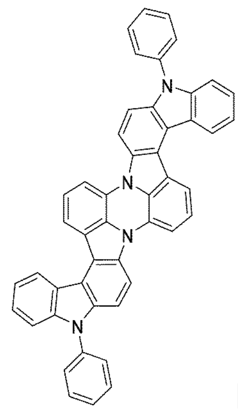
D63

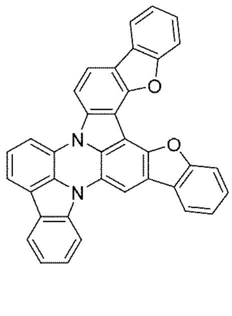
D64

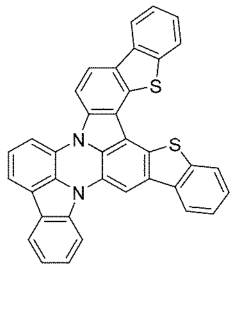
D65

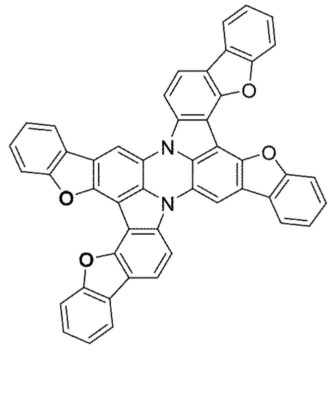
D66

[C14]

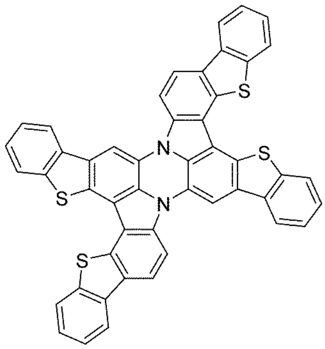
D67

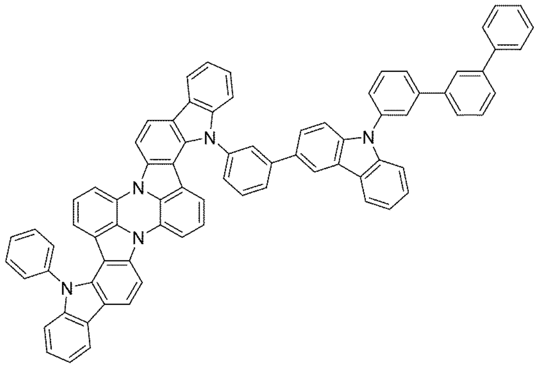
D68

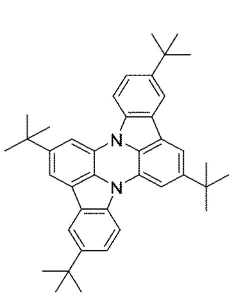
D69

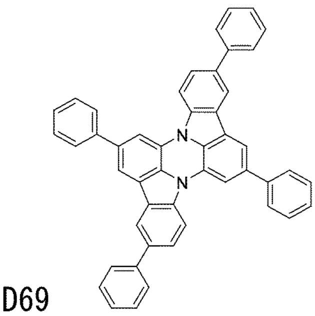
D70

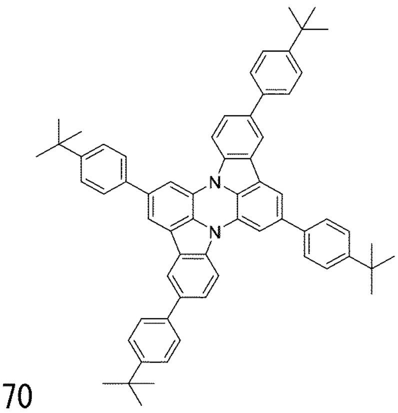
D71

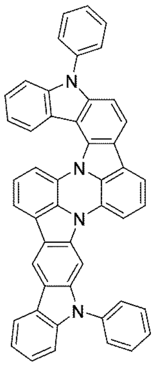
D72

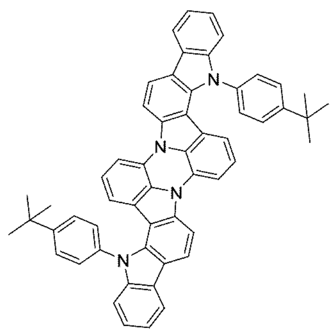
D73

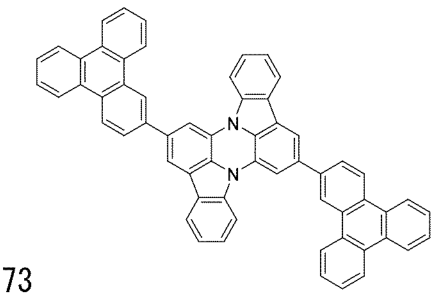
D74

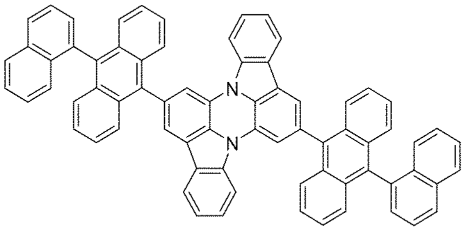
D75

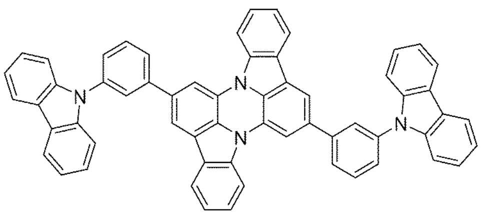
D76

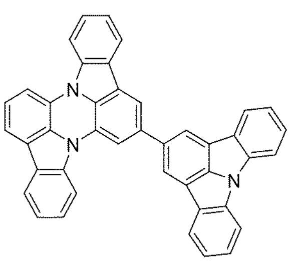
D77

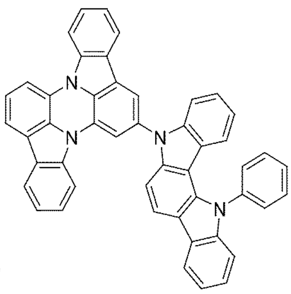
D78

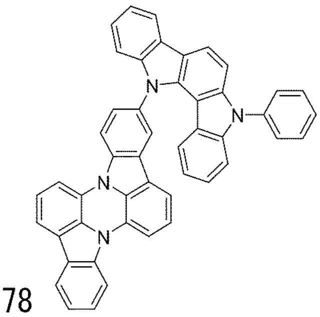
D79

[C15]

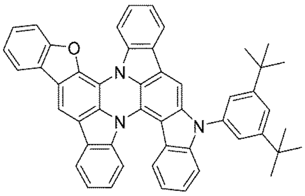
D80
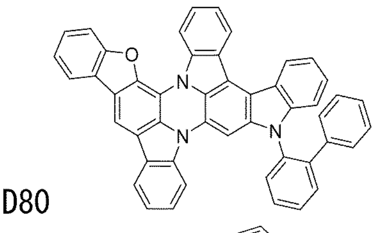
D81
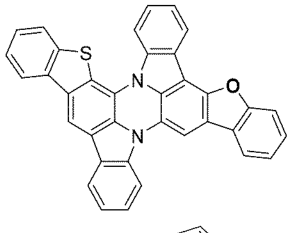
D82

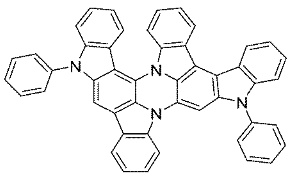
D83
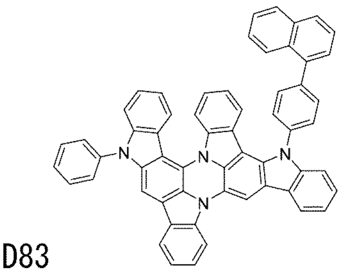
D84
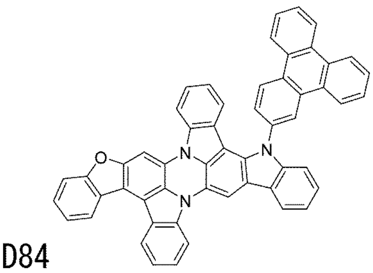
D85

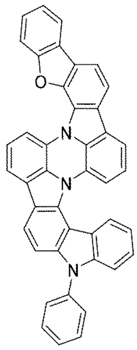
D86
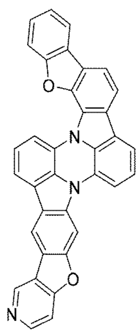
D87
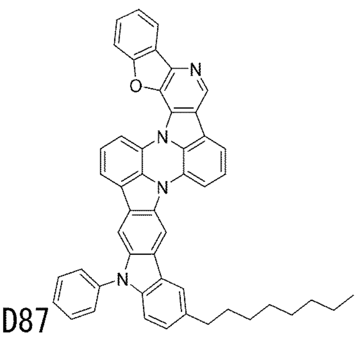
D88
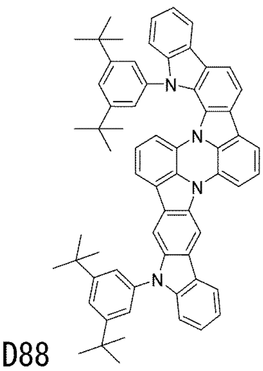
D89

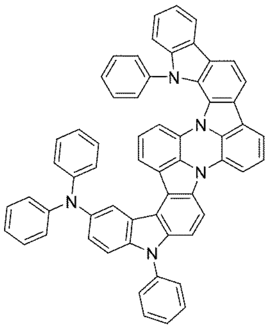
D90
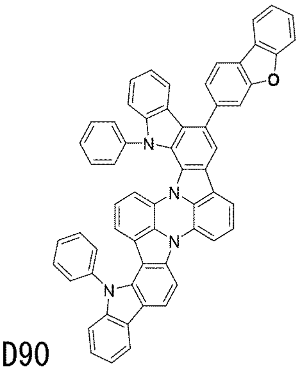
D91
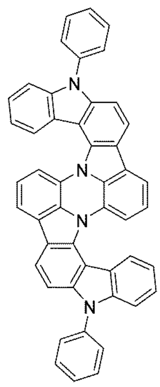
D92
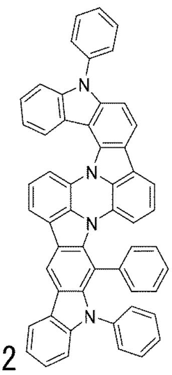
D93

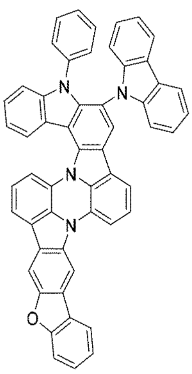
D94
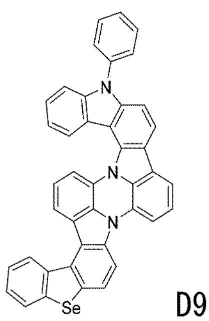
D95
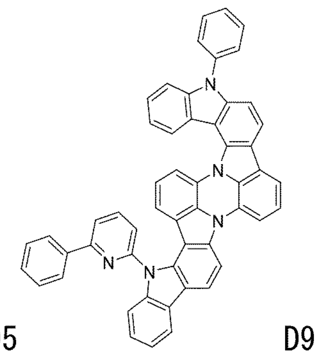
D96
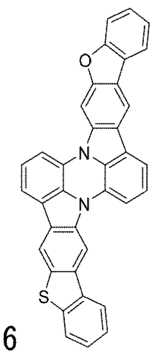
D97

[C16]

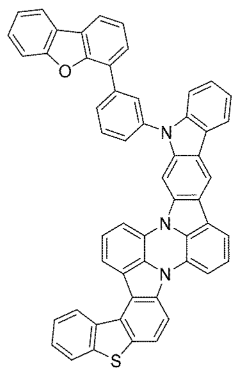
D98

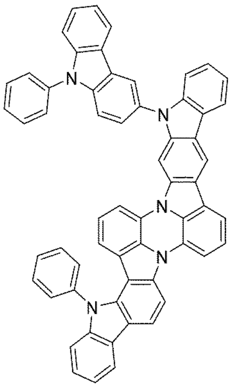
D99

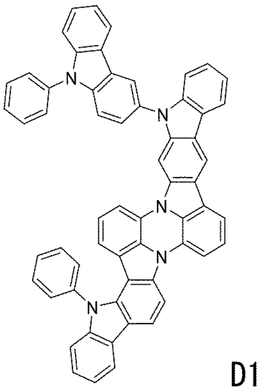
D100

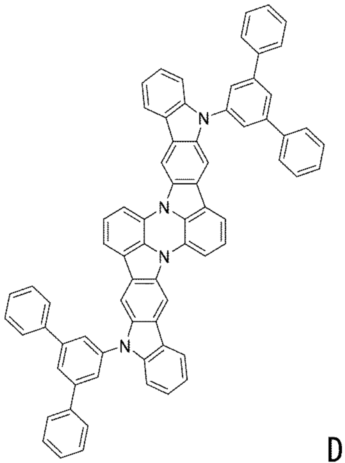
D101

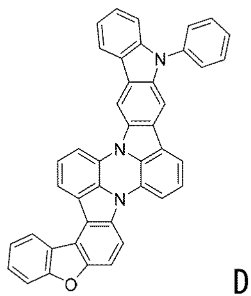
D102

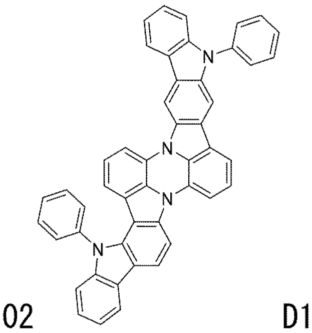
D103

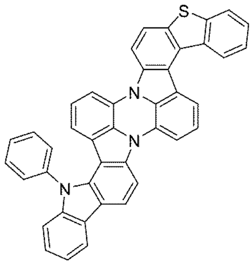
D104

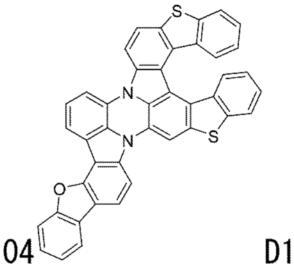
D104a

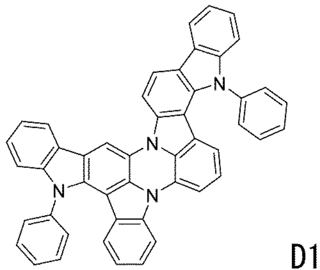
D105

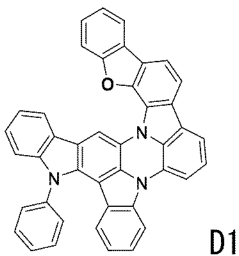
D106

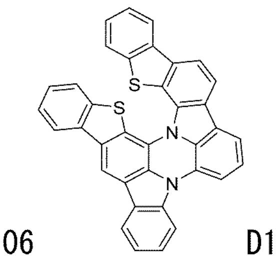
D107

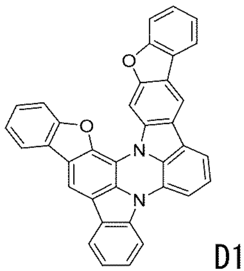
D108

[C17]

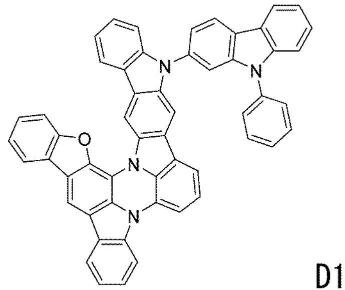

D109

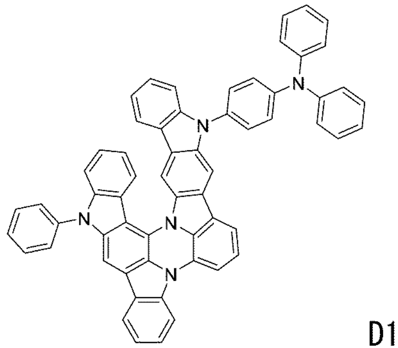

D110

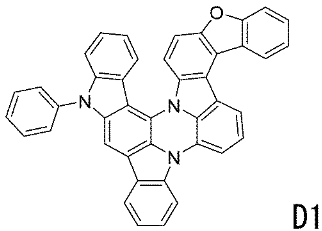

D111

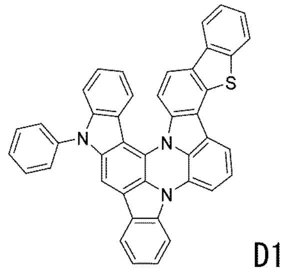

D112

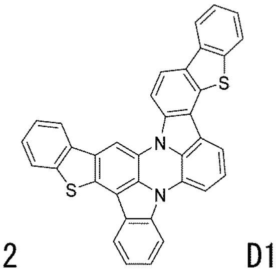

D113

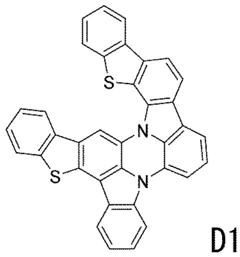

D114

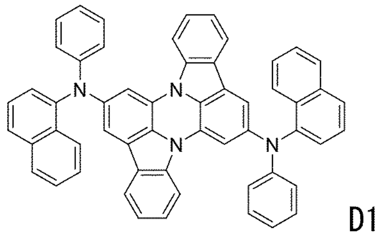

D115

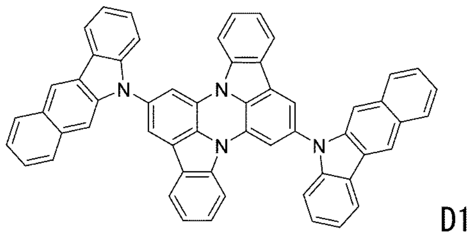

D116

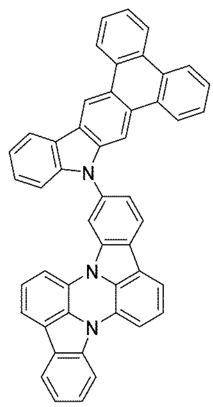

D117

[C18]

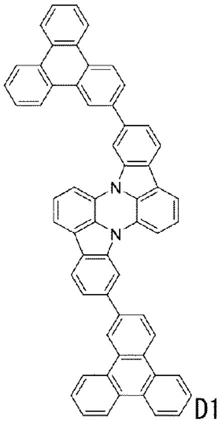
D118

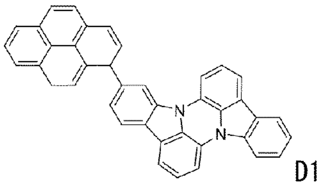
D119

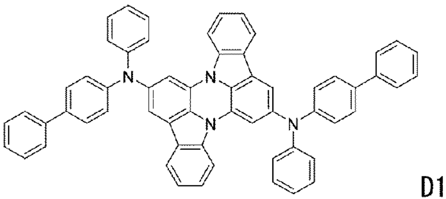
D120

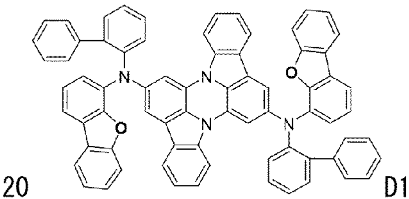
D121

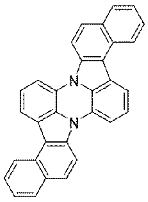
D122

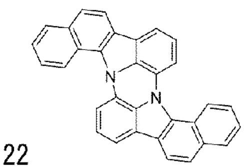
D123

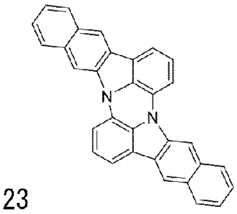
D124

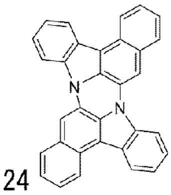
D125

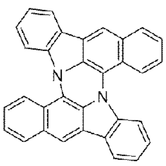
D126

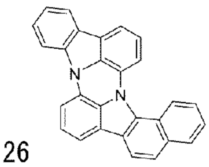
D127

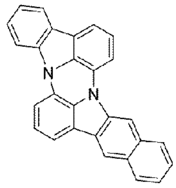
D128

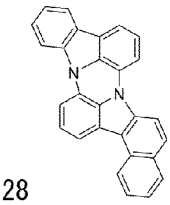
D129

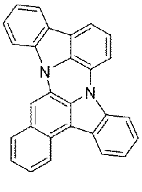
D130

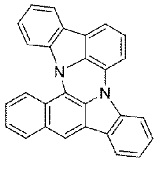
D131

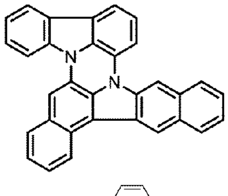
D132

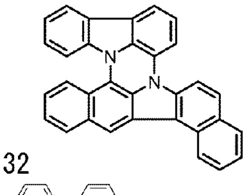
D133

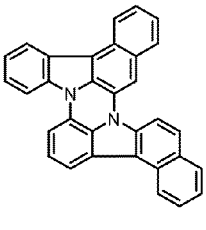
D134

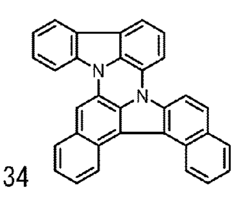
D135

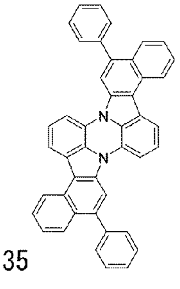
D136

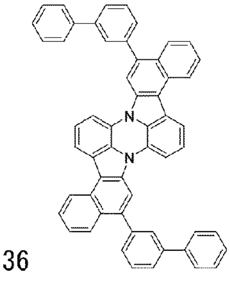
D137

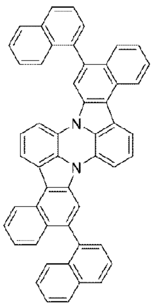 D138 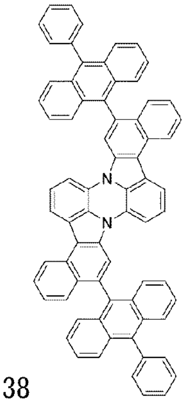 D139 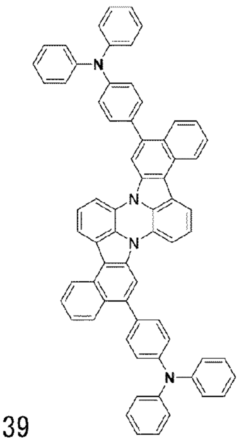 D140 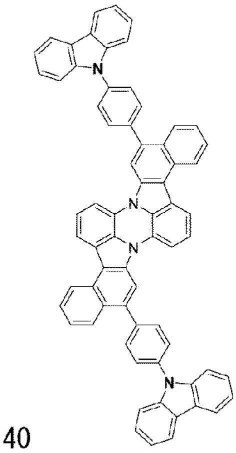 D141

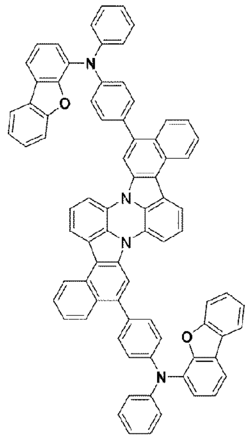 D142 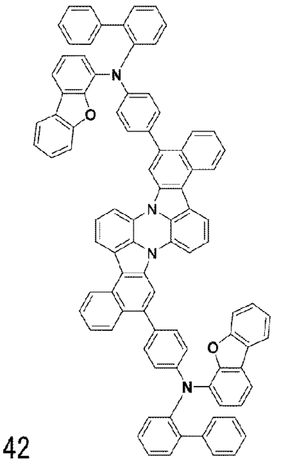 D143 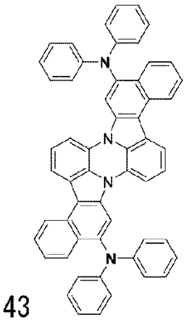 D144 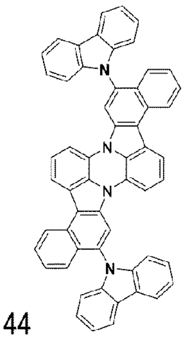 D145

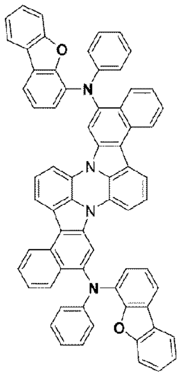 D146 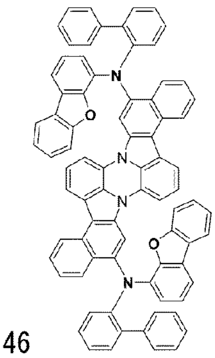 D147 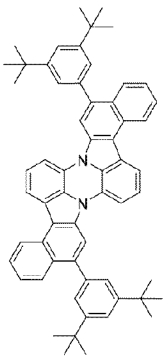 D148 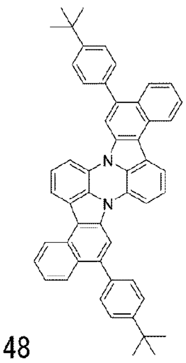 D149

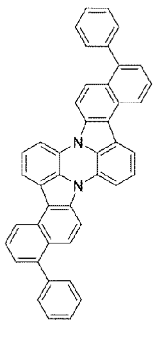 D150 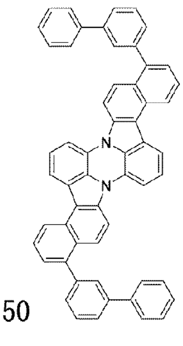 D151

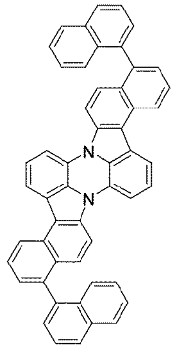
D152

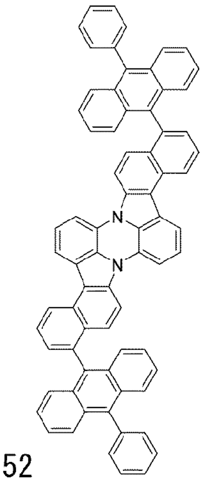
D153

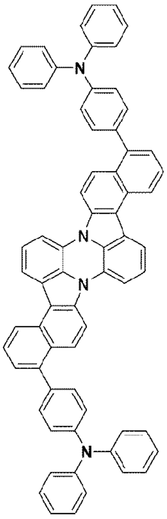
D154

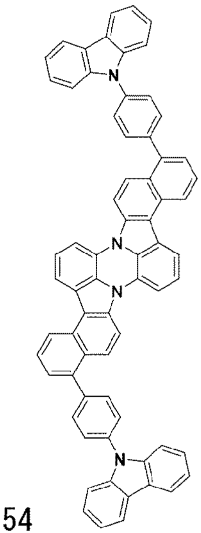
D155

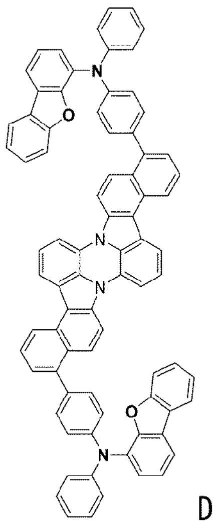
D156

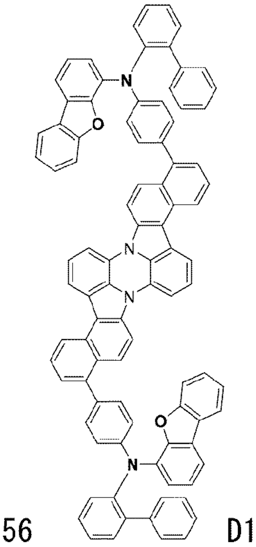
D157

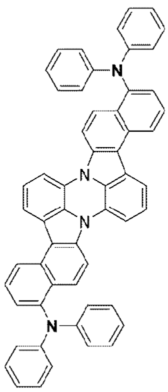
D158

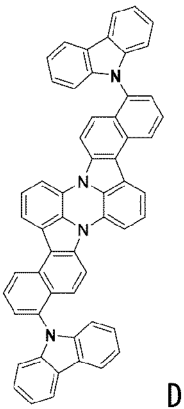
D159

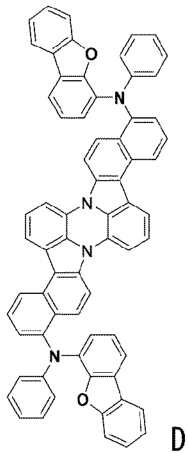
D160

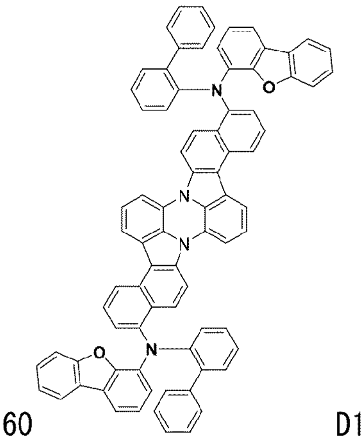
D161

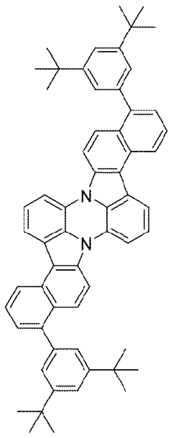
D162

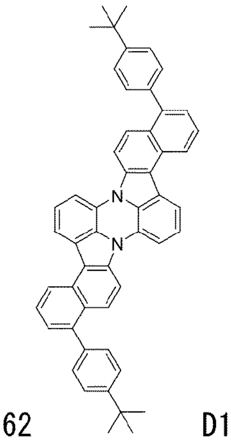
D163

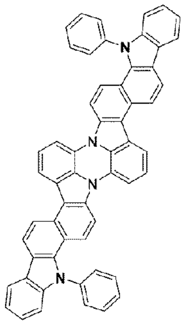
D164

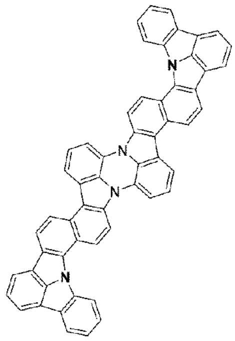
D165

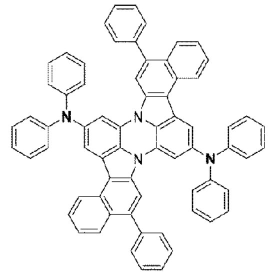
D166

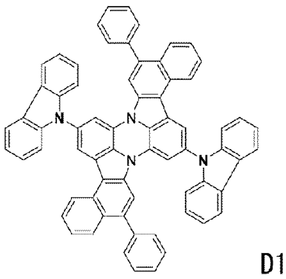
D167

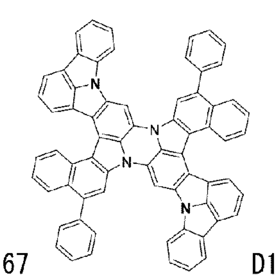
D168

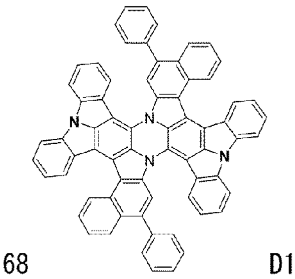
D169

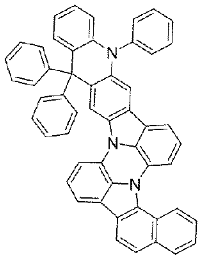
D170

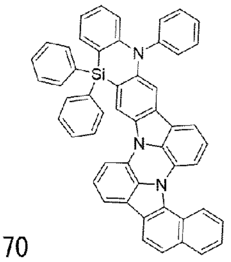
D171

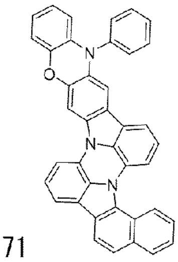
D172

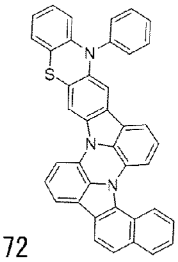
D173

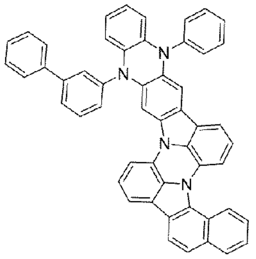
D174

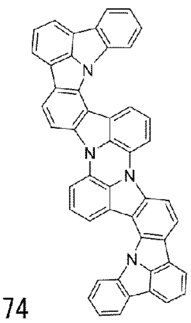
D175

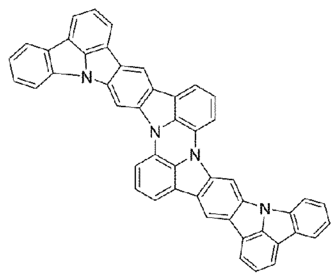
D176

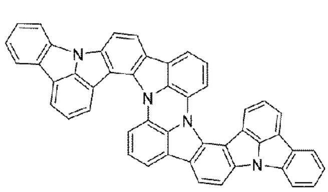
D177

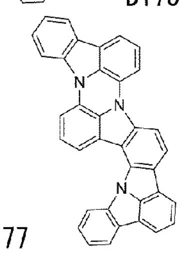
D178

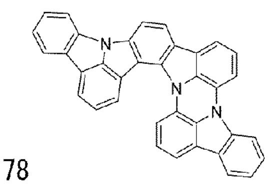
D179

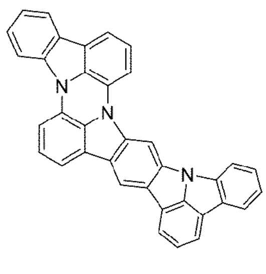
D180

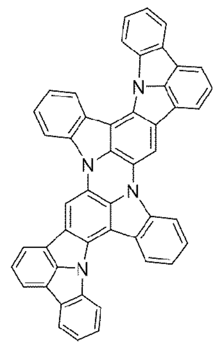
D181

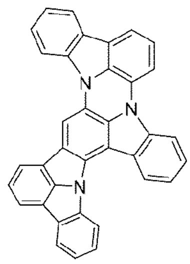
D182

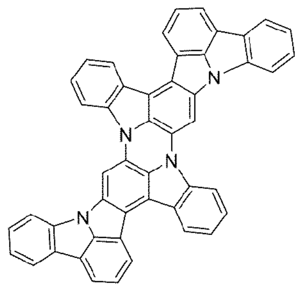
D183

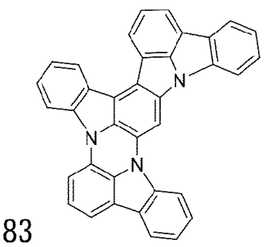
D184

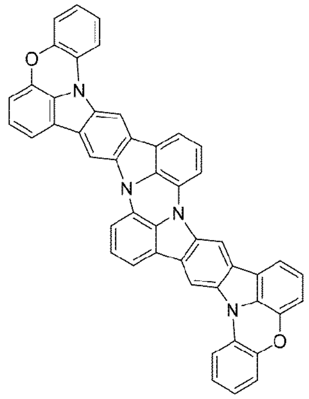
D185

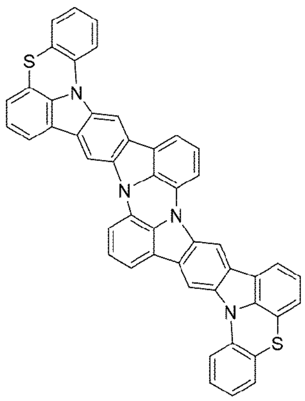
D186

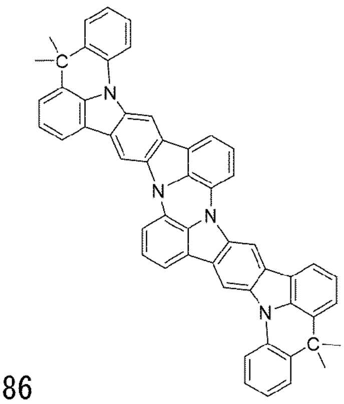
D187

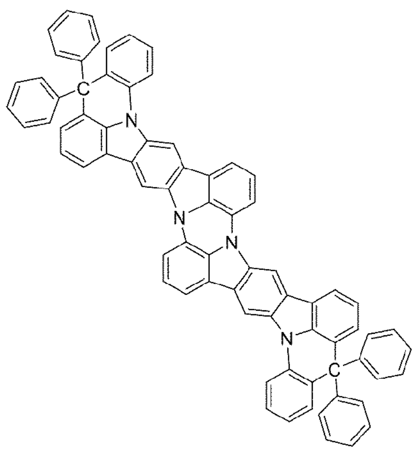
D188

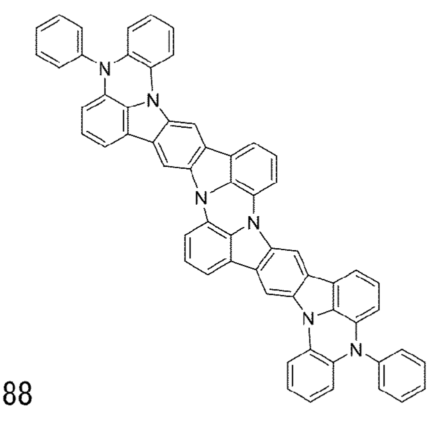
D189

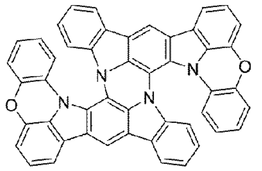 D190 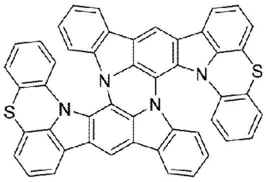 D191 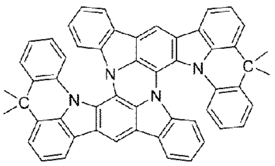 D192

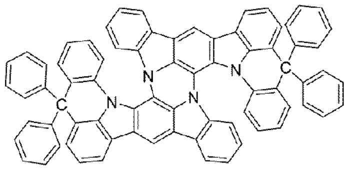 D193 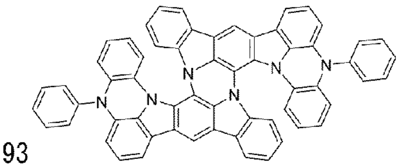 D194

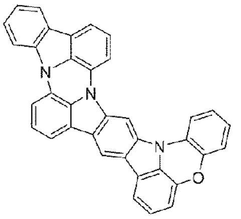 D195 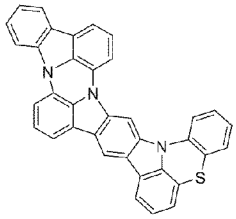 D196 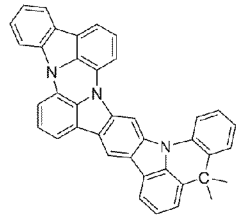 D197

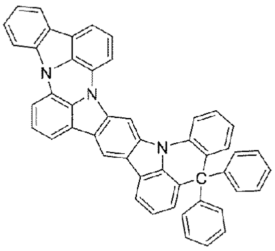 D198 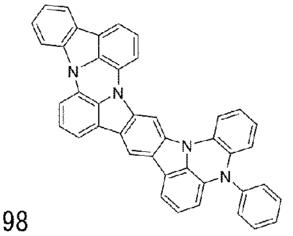 D199 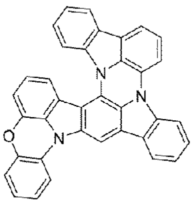 D200

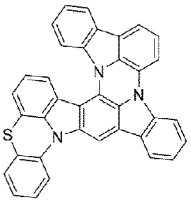 D201 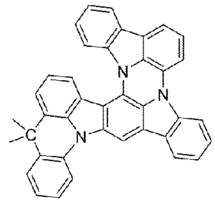 D202 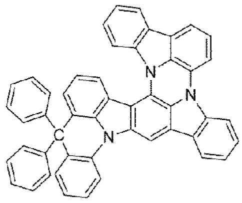 D203

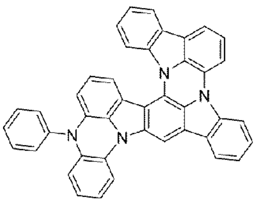 D204 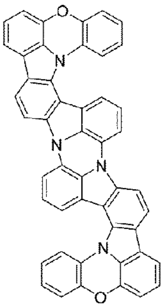 D205 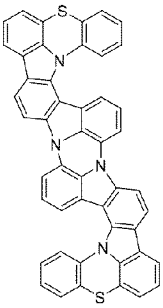 D206

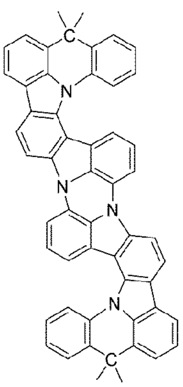
D207

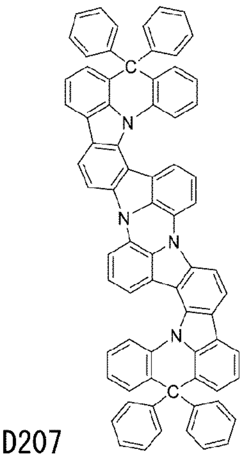
D208

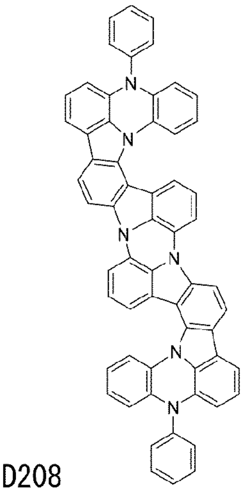
D209

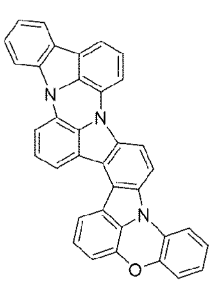
D210

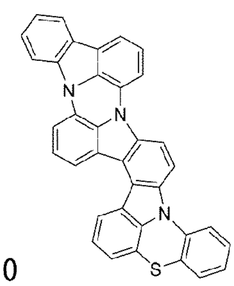
D211

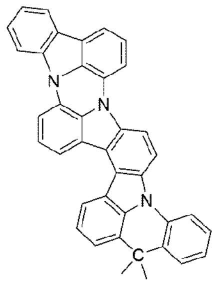
D212

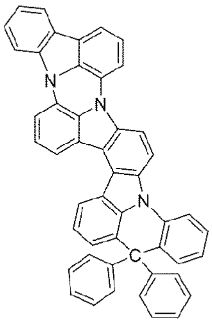
D213

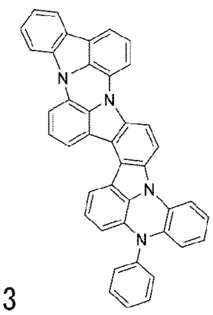
D214

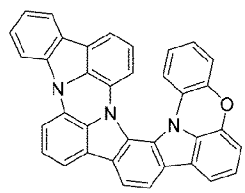
D215

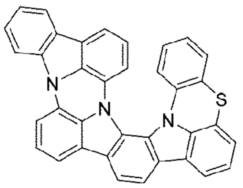
D216

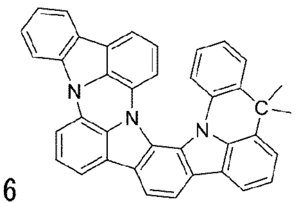
D217

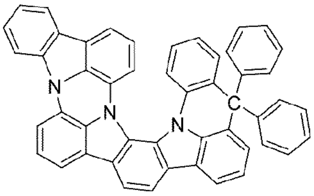
D218

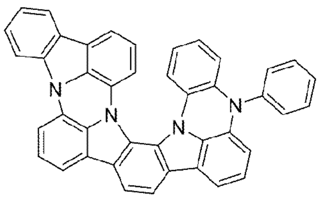
D219

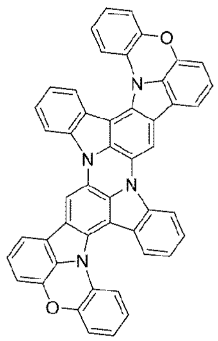
D220

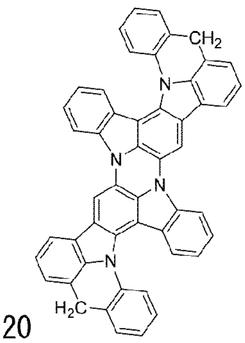
D221

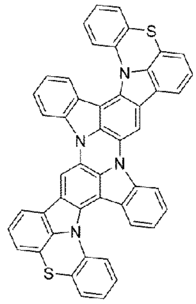
D222

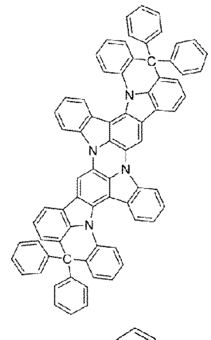
D223

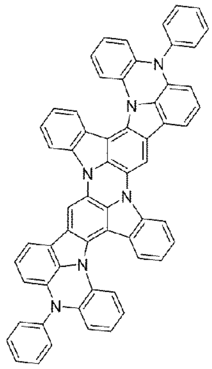
D224

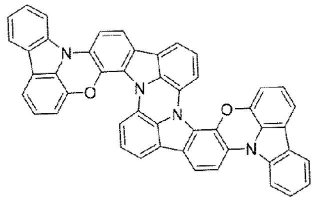
D226

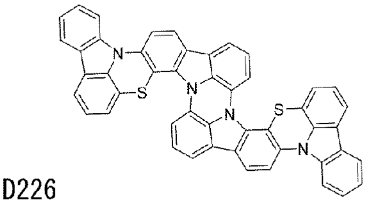
D227

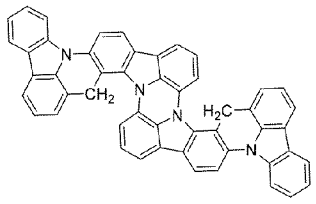
D228

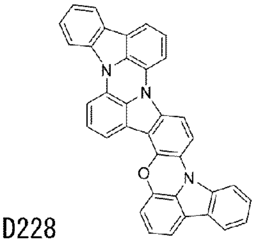
D229

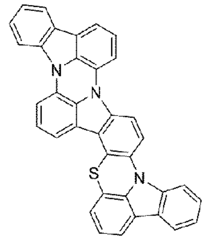
D230

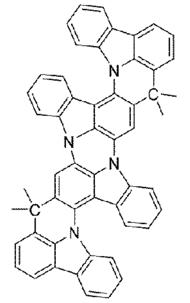
D231

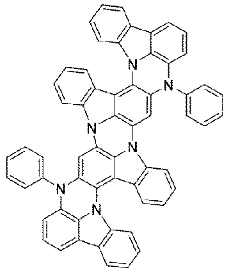
D232

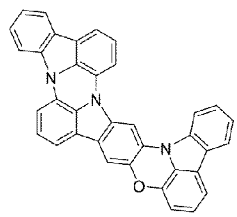
D233

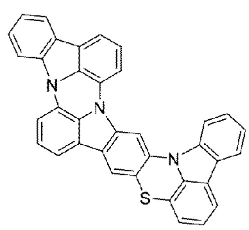
D234

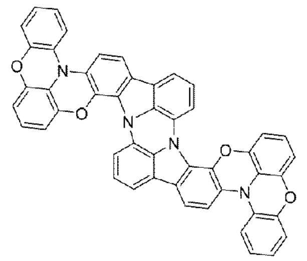
D235

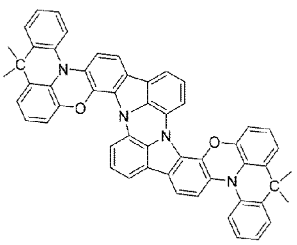
D236

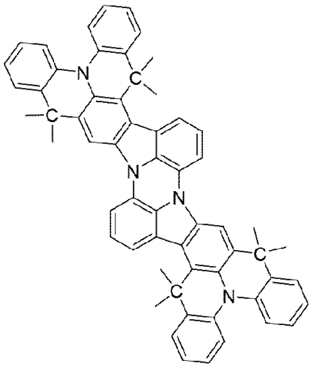
D237

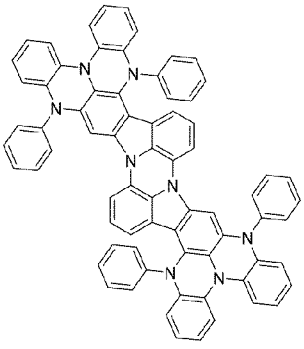
D238

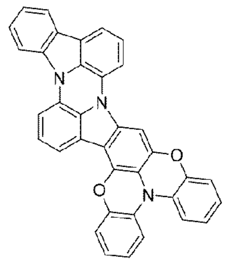
D239

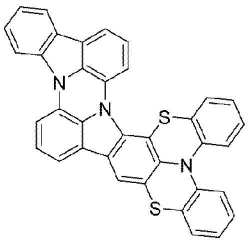
D240

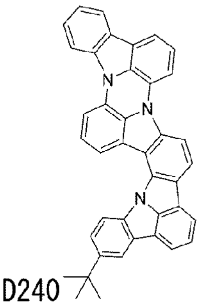
D241

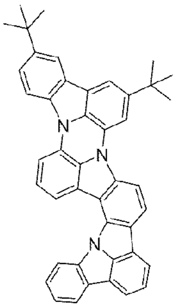
D242

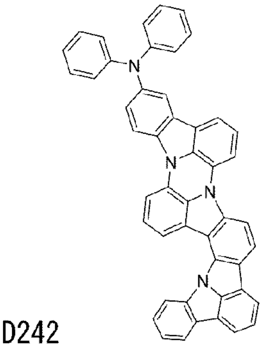
D243

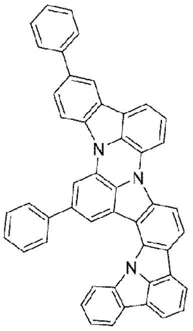
D244

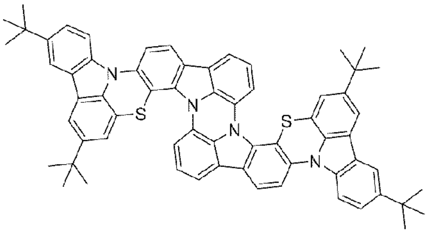
D245

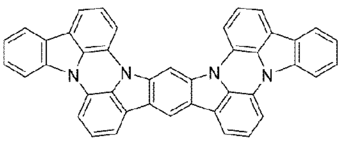
D246

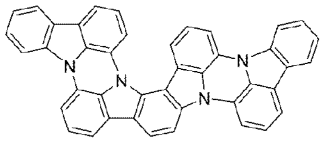
D247

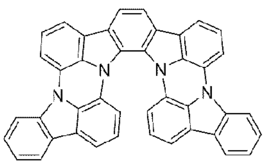
D248

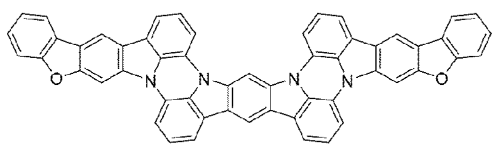
D249

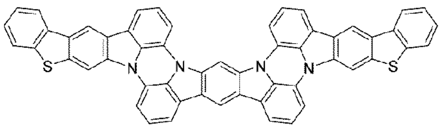
D250

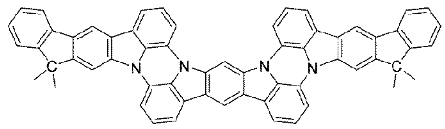
D251

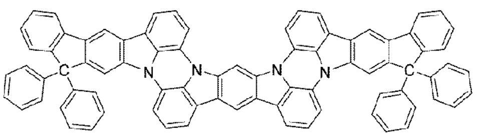
D252

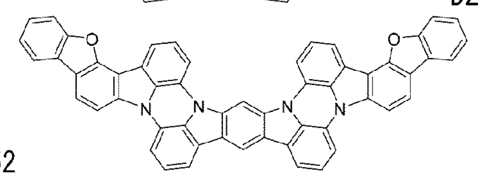
D253

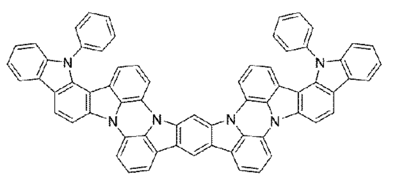
D254
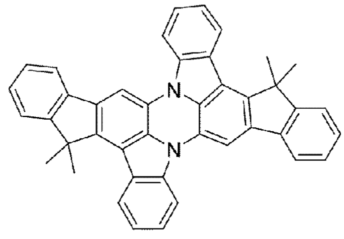
D255
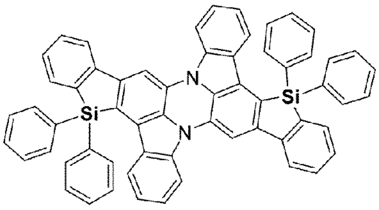
D256
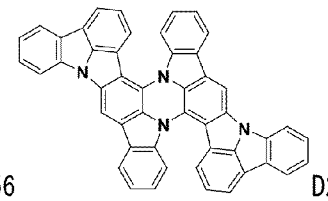
D257

By incorporating the emission material represented by the general formula (1) or general formula (1-1) into a light emitting layer, a practically excellent organic EL device having high emission efficiency and high driving stability can be provided.

Next, the structure of the organic EL device of the present invention will be described with reference to the drawing, but the structure of the organic EL device of the present invention is not limited thereto.

Figure 1 shows a cross-sectional view of a structure example of a typical organic EL device used in the present invention. Reference numeral 1 denotes a substrate, reference numeral 2 denotes an anode, reference numeral 3 denotes a hole injection layer, reference numeral 4 denotes a hole transport layer, reference numeral 5 denotes a light emitting layer, reference numeral 6 denotes an electron transport layer, and reference numeral 7 denotes a cathode. The organic EL device of the present invention may have an exciton blocking layer adjacent to the light emitting layer, or may have an electron blocking layer between the light emitting layer and the hole injection layer. The exciton blocking layer may be inserted on either the cathode side or the anode side of the light emitting layer or may be inserted on both sides at the same time. The organic EL device of the present invention has the anode, the light emitting layer, and the cathode as essential layers, but preferably has a hole injection/transport layer and an electron injection/transport layer in addition to the essential layers, and further preferably has a hole blocking layer between the light emitting layer and the electron injection/transport layer. The hole injection/transport layer means either or both of the hole injection layer and the hole transport layer, and the electron injection/transport layer means either or both of the electron injection layer and electron transport layer.

It is also possible to have a structure that is the reverse of the structure shown in Figure 1, that is, the cathode 7, the electron transport layer 6, the light emitting layer 5, the hole transport layer 4, the hole injection layer 3, and the anode 2 can be laminated on the substrate 1, in the order presented. Also, in this case, layers can be added or omitted, as necessary. In the organic EL device as described above, layers other than electrodes such as an anode and a cathode, the layers constituting a multilayer structure on a substrate, may be collectively referred to as an organic layer in some cases.

-Substrate-

The organic EL device of the present invention is preferably supported on a substrate. The substrate is not particularly limited and may be a substrate conventionally used for organic EL devices, and for example, a substrate made of glass, transparent plastic, or quartz can be used.

-Anode-

As the anode material in the organic EL device, a material made of a metal, alloy, or conductive compound having a high work function (4 eV or more), or a mixture thereof is preferably used. Specific examples of such an electrode material include metals such as Au, and conductive transparent materials such as CuI, indium tin oxide (ITO), $SnO_2$, and ZnO. An amorphous material capable of producing a transparent conductive film such as IDIXO ($In_2O_3$-ZnO) may also be used. As the anode, these electrode materials may be formed into a thin film by a method such as vapor deposition or sputtering, and then a pattern of a desired form may be formed by photolithography. Alternatively, when a highly precise pattern is not required (about 100 μm or more), a pattern may be formed through a mask of a desired form at the time of vapor deposition or sputtering of the above electrode materials. Alternatively, when a coatable material such as an organic conductive compound is used, a wet film forming method such as a printing method and a coating method

can also be used. When light is extracted from the anode, the transmittance is desirably more than 10%, and the sheet resistance as the anode is preferably several hundred Ω/square or less. The film thickness is selected within a range of usually 10 to 1,000 nm, and preferably 10 to 200 nm, although it depends on the material.

-Cathode-

On the other hand, a material made of a metal (referred to as an electron injection metal), alloy, or conductive compound having a low work function (4 eV or less) or a mixture thereof is used as the cathode material. Specific examples of such an electrode material include sodium, a sodium-potassium alloy, magnesium, lithium, a magnesium/copper mixture, a magnesium/silver mixture, a magnesium/aluminum mixture, a magnesium/indium mixture, an aluminum/aluminum oxide ($Al_2O_3$) mixture, indium, a lithium/aluminum mixture, and a rare earth metal. Among them, in terms of electron injection properties and durability against oxidation and the like, a mixture of an electron injection metal with a second metal that has a higher work function value than the electron injection metal and is stable, for example, a magnesium/silver mixture, a magnesium/aluminum mixture, a magnesium/indium mixture, an aluminum/aluminum oxide ($Al_2O_3$) mixture, a lithium/aluminum mixture, or aluminum is suitable. The cathode can be produced by forming a thin film from these cathode materials by a method such as vapor deposition and sputtering. The sheet resistance as the cathode is preferably several hundred Ω/square or less, and the film thickness is selected within a range of usually 10 nm to 5 μm, and preferably 50 to 200 nm. To transmit the light emitted, either one of the anode and the cathode of the organic EL device is favorably transparent or translucent because light emission brightness is improved.

The above metal is formed on a cathode to have a film thickness of 1 to 20 nm, and then a conductive transparent material mentioned in the description of the anode is formed on the metal, so that a transparent or translucent cathode can be produced. By applying this process, a device in which both anode and cathode have transmittance can be produced.

-Light Emitting Layer-

The light emitting layer is a layer that emits light after holes and electrons respectively injected from the anode and the cathode are recombined to form exciton. For the light emitting layer, the emission material represented by the general formula (1) or general formula (1-1) may be used alone, or the emission material may be used in combination with a host material. When the emission material is used together with the host material, the emission material serves to emit light in a device.

The content of the emission material is preferably 0.1 to 50 wt%, and more preferably 0.1 to 40 wt% based on the host material.

The host material in the light emitting layer can be a known host material used for a phosphorescent device or a fluorescent device. A usable known host material is a compound having the ability to transport hole, the ability to transport electron, and a high glass transition temperature, and preferably has a higher triplet excited energy (T1) than the triplet excited energy (T1) of the emission material represented by the general formula (1). A TADF-active compound may also be used as the host material, and in that case, the compound preferably has a difference (ΔEST = S1 - T1) between the singlet excited energy (S1) and the triplet excited energy (T1), of 0.20 eV or less.

Such host materials are known in a large number of Patent Literatures and the like, and hence may be selected from them. Specific examples of the host material include, but are not particularly limited to, various metal complexes typified by metal complexes of indole compounds, carbazole compounds, indolocarbazole compounds, pyridine compounds, pyrimidine compounds, triazine compounds, triazole compounds, oxazole compounds, oxadiazole compounds, imidazole compounds, phenylenediamine compounds, arylamine compounds, anthracene compounds, fluorenone compounds, stilbene compounds, triphenylene compounds, carborane compounds, porphyrin compounds, phthalocyanine compounds, and 8-quinolinol compounds, and metal phthalocyanine, and metal complexes of benzoxazole and benzothiazole compounds; and polymer compounds such as poly(N-vinyl carbazole) compounds, aniline-based copolymer compounds, thiophene oligomers, polythiophene compounds, polyphenylene compounds, polyphenylene vinylene compounds, and polyfluorene compounds. Preferred examples thereof include carbazole compounds, indolocarbazole compounds, pyridine compounds, pyrimidine compounds, triazine compounds, anthracene compounds, triphenylene compounds, carborane compounds, and porphyrin compounds.

Only one host may be contained or two or more hosts may be used in one light emitting layer. When two or more hosts are used, at least one thereof is preferably an electron-transporting compound, for example, the triazine compounds or anthracene compounds described above, and other host is preferably a hole-transporting compound, for example, the carbazole compounds or indolocarbazole compounds. When a plurality of hosts is used, each host is deposited from different deposition sources, or a plurality of hosts is premixed before vapor deposition to form a premix, whereby a plurality of hosts can be simultaneously deposited from one deposition source.

The emission material and the host material can be respectively deposited from different deposition sources,

or can be premixed before vapor deposition to form a premix, whereby the emission material and the host material can be simultaneously deposited from one deposition source.

As the method of premixing, a method by which hosts can be mixed as uniformly as possible is desirable, and examples thereof include, but are not limited to, milling, a method of heating and melting hosts under reduced pressure or under an inert gas atmosphere such as nitrogen, and sublimation.

The host and a premix thereof may be in the form of powder, sticks, or granules.

-Injection Layer-

The injection layer refers to a layer provided between the electrode and the organic layer to reduce the driving voltage and improve the light emission brightness, and includes the hole injection layer and the electron injection layer. The injection layer may be present between the anode and the light emitting layer or the hole transport layer, as well as between the cathode and the light emitting layer or the electron transport layer. The injection layer may be provided as necessary.

-Hole Blocking Layer-

The hole blocking layer has the function of the electron transport layer in a broad sense, is made of a hole blocking material having a very small ability to transport holes while having the function of transporting electrons, and can improve the recombination probability between the electrons and the holes in the light emitting layer by blocking the holes while transporting the electrons. For the hole blocking layer, a known hole blocking material can be used. A plurality of hole blocking materials may be used in combination.

-Electron Blocking Layer-

The electron blocking layer has the function of the hole transport layer in a broad sense, and can improve the recombination probability between the electrons and the holes in the light emitting layer by blocking the electrons while transporting the holes. As the material for the electron blocking layer, a known material for the electron blocking layer can be used.

-Exciton Blocking Layer-

The exciton blocking layer is a layer to block the diffusion of the excitons generated by recombination of the holes and the electrons in the light emitting layer into a charge transport layer, and insertion of this layer makes it possible to efficiently keep the excitons in the light emitting layer, so that the emission efficiency of the device can be improved. The exciton blocking layer can be inserted between two light emitting layers adjacent to each other in the device in which two or more light emitting layers are adjacent to each other. As the material for such an exciton blocking layer, a known material for the exciton blocking layer can be used.

The layer adjacent to the light emitting layer includes the hole blocking layer, the electron blocking layer, and the exciton blocking layer, and when these layers are not provided, the adjacent layer is the hole transport layer, the electron transport layer, and the like.

-Hole Transport Layer-

The hole transport layer is made of a hole transport material having the function of transporting holes, and the hole transport layer may be provided as a single layer or a plurality of layers.

The hole transport material has any of hole injection properties, hole transport properties, or electron barrier properties, and may be either an organic material or an inorganic material. As the hole transport layer, any of conventionally known compounds may be selected and used. Examples of such a hole transport material include porphyrin derivatives, arylamine derivatives, triazole derivatives, oxadiazole derivatives, imidazole derivatives, polyarylalkane derivatives, phenylenediamine derivatives, arylamine derivatives, amino-substituted chalcone derivatives, oxazole derivatives, styrylanthracene derivatives, fluorenone derivatives, hydrazone derivatives, stilbene derivatives, silazane derivatives, aniline-based copolymers, and conductive polymer oligomers, particularly, thiophene oligomers. Porphyrin derivatives, arylamine derivatives, and styrylamine derivatives are preferably used, and arylamine compounds are more preferably used.

-Electron Transport Layer-

The electron transport layer is made of a material having the function of transporting electrons, and the electron transport layer may be provided as a single layer or a plurality of layers.

The electron transport material (may also serve as the hole blocking material) has the function of transmitting electrons injected from the cathode to the light emitting layer. As the electron transport layer, any of conventionally known compounds may be selected and used, and examples thereof include polycyclic aromatic derivatives such as naphthalene, anthracene, and phenanthroline, tris(8-quinolinolato)aluminum (III) derivatives, phosphine oxide derivatives, nitro-substituted fluorene derivatives, diphenylquinone derivatives, thiopyran dioxide derivatives, carbodiimides, fluorenylidene methane derivatives, anthraquinodimethane and anthrone derivatives, bipyridine derivatives, quinoline derivatives, oxadiazole derivatives, benzimidazole derivatives, benzothiazole derivatives, and indolocarbazole derivatives. Further, polymer materials in which these materials are introduced in the polymer chain or these materials constitute the main chain of the polymer can also be used.

When the organic EL device of the present invention is produced, the film formation method of each layer is not particularly limited, and the layers may be produced by either a dry process or a wet process.

S1 and T1 are measured as follows. A sample compound (thermally activated delayed fluorescence material) is deposited on a quartz substrate by a vacuum deposition method under conditions of a degree of vacuum of $10^{-4}$ Pa or less to form a deposition film having a thickness of 100 nm. For S1, the emission spectrum of this deposition film is measured, a tangent is drawn to the rise of the emission spectrum on the short-wavelength side, and the wavelength value λedge [nm] of the point of intersection of the tangent and the horizontal axis is substituted into the following equation (i) to calculate S1.

$$S1\ [eV] = 1239.85/\lambda edge \qquad (i)$$

For T1, on the other hand, the phosphorescence spectrum of the above deposition film is measured, a tangent is drawn to the rise of the phosphorescence spectrum on the short-wavelength side, and the wavelength value λedge [nm] of the point of intersection of the tangent and the horizontal axis is substituted into the following equation (ii) to calculate T1.

$$T1\ [eV] = 1239.85/\lambda edge \qquad (ii)$$

Examples

Synthesis Example 1

[C19]

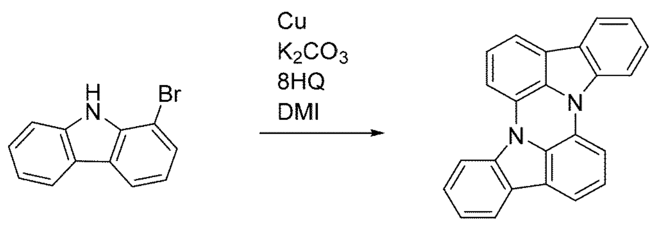


(A) (D1)

In a three-necked flask were put 3.0 g of a starting material (A), 0.78 g of copper, 3.5 g of potassium carbonate, 0.36 g of 8-quinolinol (8HQ), and 6.0 ml of 1,3-dimethyl-2-imidazolidinone (DMI) under a nitrogen atmosphere, and stirred at 190°C for 72 hours. The reaction solution was cooled to room temperature, and methanol was added in small amounts to take the resulting precipitate by filtering. The product taken by filtering was purified by silica gel column chromatography. Thereafter, the product was washed with methanol, and the resulting solid was dried under reduced pressure to yield 0.15 g of a compound (D1) (yield: 7.2%).
APCI-TOFMS m/z 331[M+1]

Synthesis Example 2

[C20]

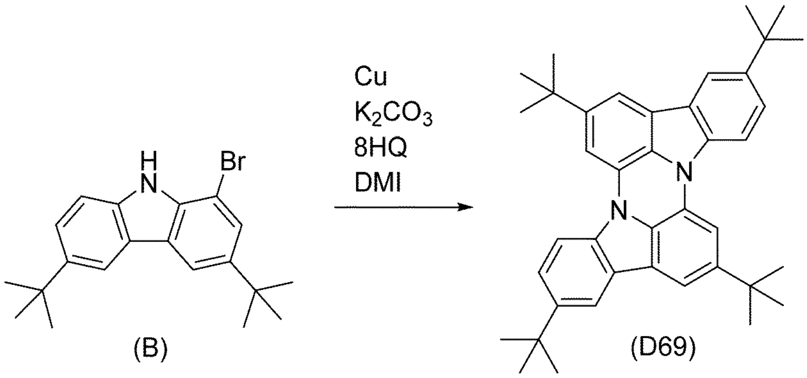


(B) (D69)

In a three-necked flask were put 3.0 g of a starting material (B), 0.53 g of copper, 2.4 g of potassium carbonate, 0.25 g of 8-quinolinol (8HQ), and 4.0 ml of 1,3-dimethyl-2-imidazolidinone (DMI) under a nitrogen atmosphere, and stirred at 190°C for 72 hours. The reaction solution was cooled to room temperature, and methanol was added in small amounts to take the resulting precipitate by filtering. The product taken by filtering was purified by silica gel column chromatography. Thereafter, the product was washed with methanol, and the resulting solid was dried under reduced pressure to yield 0.4 g of a compound (D69) (yield: 9.2%).

APCI-TOFMS m/z 554[M+1]

Synthesis Example 3

[C21]

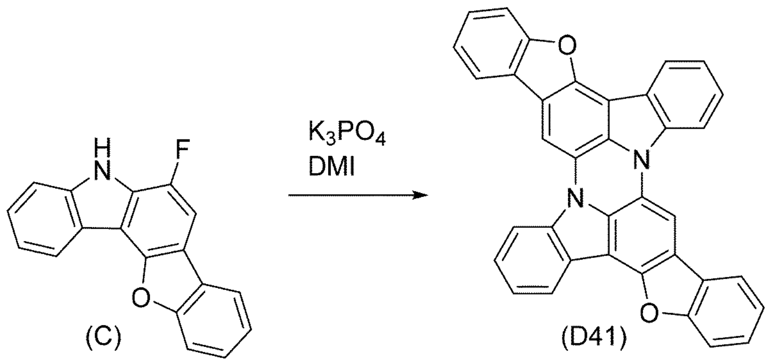


(C) (D41)

In a three-necked flask were put 5.6 g of a starting material (C), 12.9 g of tripotassium phosphate, and 48.0 ml of 1,3-dimethyl-2-imidazolidinone (DMI) under a nitrogen atmosphere, and stirred at 230°C for 14 days. The reaction solution was cooled to room temperature, and methanol was added in small amounts to take the resulting precipitate by filtering. The product taken by filtering was purified by re-precipitation by tetrahydrofuran and xylene. Thereafter, the product was washed with methanol, and the resulting solid was dried under reduced pressure to yield 0.4 g of a compound (D41) (yield: 4.0%).

APCI-TOFMS m/z 510[M+1]

Synthesis Example 4

[C22]

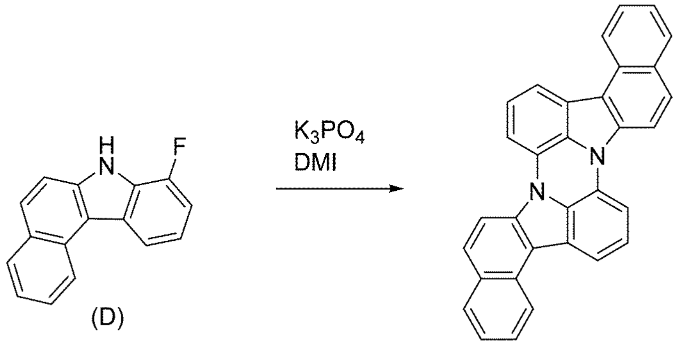


(D122)

In a three-necked flask were put 7.8 g of a starting material (D), 21.1 g of tripotassium phosphate, and 83.0 ml of 1,3-dimethyl-2-imidazolidinone (DMI) under a nitrogen atmosphere, and stirred at 230°C for 10 days. The reaction solution was cooled to room temperature, and methanol was added in small amounts to take the resulting precipitate by filtering. The product taken by filtering was purified by re-precipitation by tetrahydrofuran and xylene. Thereafter, the product was washed with methanol, and the resulting solid was dried under reduced pressure to yield 0.5 g of a compound (D122) (yield: 3.5%).

APCI-TOFMS m/z 430[M+1]

Example 1

The following thin film was formed on a quartz substrate by a vacuum deposition method at a degree of vacuum of $4.0 \times 10^{-5}$ Pa. BH-1 as the host and the compound (D1) as the dopant were co-deposited from different deposition sources to form a light emitting layer having a thickness of 100 nm. At this time, they were co-deposited under deposition conditions such that the concentration of the compound (D1) was 2% by mass. Each organic thin film according to Example 1 was produced.

The photoluminescence quantum yield (PLQY) of each organic thin film described above was measured with Absolute PL Quantum Yield Measurement C9920-03G system (Hamamatsu Photonics K.K.). C9920-03G system can be used to thereby continuously measure the photoexcitation and emission spectra of each organic thin film, and the energy balance here can be calculated to thereby calculate PLQY of each organic thin film. The maximum emission wavelength, the half-value width, PLQY and CIE coordinates can be determined with software U6039-05 version 3.6.0. The maximum emission wavelength and the half-value width are given as values of nm, PLQY is given as a value of %, and CIE coordinates are given as x and y values. The excitation wavelength in PLQY measurement was 340 nm.

Example 2

Each organic thin film according to Example 2 was produced in the same manner as in Example 1, except that the compound (D69) was used as the dopant.

Example 3

A solution of the compound (D41) in toluene, in which the concentration was adjusted to $5 \times 10^{-6}$ M was produced. The maximum emission wavelength, the half-value width, PLQY and CIE coordinates with respect to the

solution were determined in the same manner as in Example 1.

Example 4

A solution of the compound (D122) in toluene, in which the concentration was adjusted to 5 × $10^{-6}$ M, was produced. The maximum emission wavelength, the half-value width, PLQY and CIE coordinates with respect to the solution were determined in the same manner as in Example 1.

Comparative Example 1

Each organic thin film was produced in the same manner as in Example 1, except that the dopant was changed to BH-1.

The compounds used in Examples and Comparative Examples are shown below.

[C23]

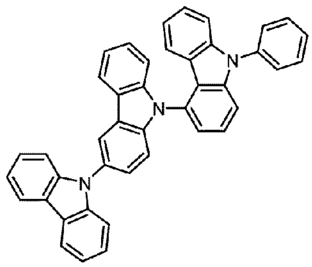

(BH-1)

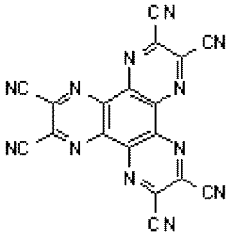

HAT-CN

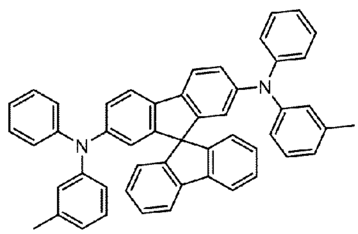

(HT-1)

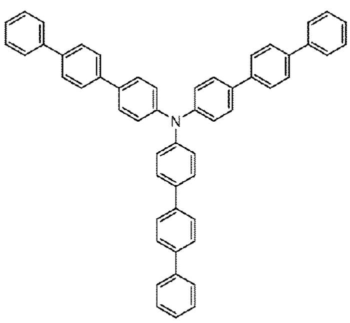

(HT-2)

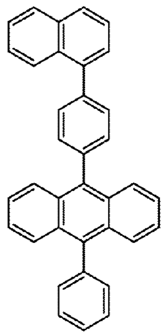

(BH-2)

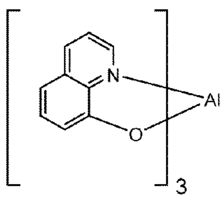

(ALQ3)

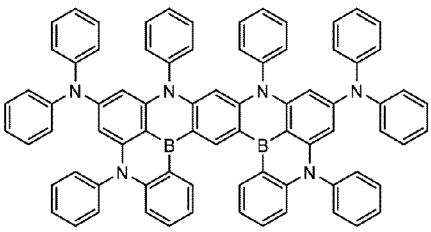

ν-DABNA

The measurement results of the maximum emission wavelength of the emission spectrum, the half-value width, the chromaticity (CIEx, CIEy), and PLQY of each of the produced organic thin films and solutions are shown in Table 1.

[Table 1]

| | Maximum emission wavelength (nm) | Half-value width (nm) | CIEx | CIEy | PLQY (%) |
|---|---|---|---|---|---|
| Example 1 | 431 | 42 | 0.16 | 0.07 | 70 |
| Example 2 | 443 | 46 | 0.16 | 0.08 | 74 |
| Example 3 | 452 | 16 | 0.17 | 0.12 | 71 |
| Example 4 | 456 | 19 | 0.14 | 0.15 | 79 |
| Comparative Example 1 | 418 | 48 | 0,17 | 0.05 | 37 |

The emission material of the present invention is found from Table 1 to have high efficiency characteristics and is found from the maximum emission wavelength to exhibit blue light emission.

Example 5

Each thin film shown below was laminated on the glass substrate on which an anode made of ITO having a film thickness of 70 nm was formed by a vacuum deposition method at a degree of vacuum of 4.0 × 10-5 Pa. First, the previously presented HAT-CN was formed on ITO to a thickness of 10 nm as a hole injection layer, and then HT-1 was formed to a thickness of 25 nm as a hole transport layer. Then, HT-2 was formed to a thickness of 5 nm as an electron blocking layer. Then, BH-2 as the host and the compound (D122) as the dopant were co-deposited from different deposition sources to form a light emitting layer having a thickness of 30 nm. At this time, they were co-deposited under deposition conditions such that the concentration of the compound (D122) was 1% by mass. Then, BH-2 was formed to a thickness of 5 nm as a hole blocking layer. Then, ALQ3 was formed to a thickness of 40 nm as an electron transport layer. Further, lithium fluoride (LiF) was formed on the electron transport layer to a thickness of 1 nm as an electron injection layer. Finally, aluminum (Al) was formed on the electron injection layer to a thickness of 70 nm as a cathode, whereby an organic EL device according to Example 5 was produced.

Comparative Example 2

Each organic EL device was produced in the same manner as in Example 5, except that v-DABNA was used as the dopant.

The maximum emission wavelength of the emission spectrum, external quantum efficiency, and lifetime of each organic EL device produced are shown in Table 2. The maximum emission wavelength and the external quantum efficiency were values at a driving current density of 2.5 $mA/cm^2$ and were initial characteristics. The time taken for the luminance to reduce to 90% of the initial luminance when the driving current density was 40 $mA/cm^2$ was measured as the lifetime.

[Table 2]

| | Maximum emission wavelength (nm) | External quantum efficiency (%) | Lifetime (h) |
|---|---|---|---|
| Example 5 | 465 | 4.0 | 150 |
| Comparative Example 2 | 470 | 5.3 | 103 |

The organic EL device including the emission material of the present invention is found from Table 2 to have high characteristics and is found from the maximum emission wavelength to exhibit blue light emission. The organic EL device also exhibits particularly excellent characteristics in terms of lifetime characteristics.

Industrial Applicability

According to the emission material of the present invention, a practically useful organic EL device having high emission efficiency and high driving stability can be obtained. The emission material of the present invention exhibits the maximum wavelength in a blue, sky blue, or green spectral region. The emission material exhibits the maximum wavelength particularly at 410 nm to 550 nm, preferably 430 nm to 495 nm. The photoluminescence quantum yield of the emission material of the present invention can reach 40% or more. The emission material of the present invention is used to thereby provide a higher-efficiency device. An organic EL device having a light emitting layer including the emission material has a high emission efficiency, and a color.

Reference Signs List

1 substrate, 2 anode, 3 hole injection layer, 4 hole transport layer, 5 light emitting layer, 6 electron transport layer, 7 cathode.

## Claims

**1.** An emission material represented by the following general formula (1) or the following general formula (1-1) :

[C1]

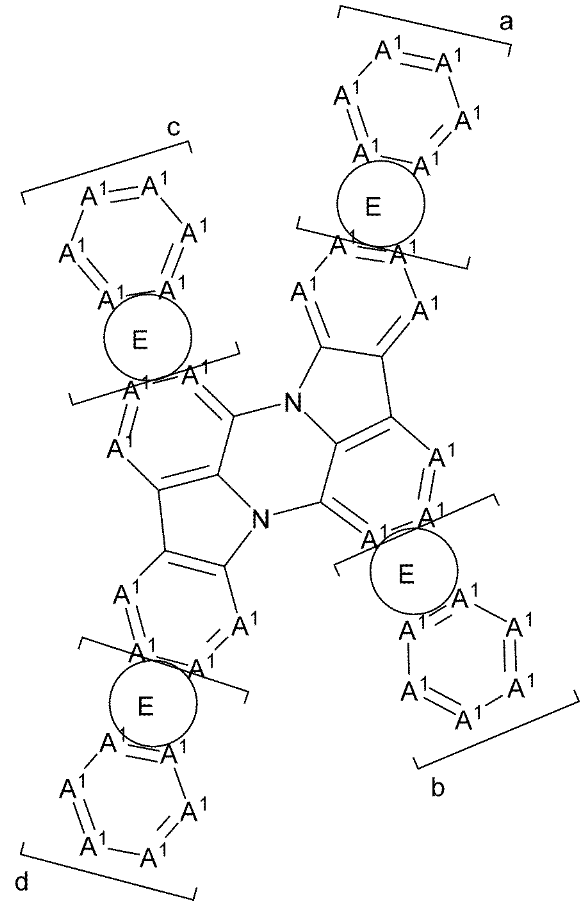

(1)

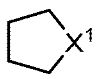

(1a)

wherein each $A^1$ independently represents $CR^1$, C or N, provided that the number of N present in one 6-membered ring containing $A^1$ in the general formula (1) is 2 or less; each $R^1$ independently represents hydrogen, a cyano group, deuterium, a substituted or unsubstituted diarylamino group having 12 to 44 carbon atoms, a substituted or unsubstituted arylheteroarylamino group having 12 to 44 carbon atoms, a substituted or unsubstituted diheteroarylamino group having 12 to 44 carbon atoms, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups; when $R^1$ represents a substituted or unsubstituted diarylamino group, arylheteroarylamino group, diheteroarylamino group or aromatic hydrocarbon group, or a substituted or unsubstituted aromatic heterocyclic group, $R^1$ optionally forms one or more bonds with an aromatic ring having $A^1$ to which $R^1$ is bonded in the formula (1), directly or via -O-, -S-, $-Si(R^a)_2-$, $-C(R^a)_2-$, or $-NR^a-$, to form a fused ring; each $R^a$ independently represents hydrogen, deuterium, an aliphatic hydrocarbon group having 1 to 10 carbon atoms, a substituted or unsubstituted aromatic hydrocarbon

group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups; each ring E independently represents a heterocycle represented by formula (1a), and

each ring E is fused with an adjacent ring at any position; each $X^1$ independently represents a group represented by $Si(R^d)_2$, $C(R^d)_2$, O, S, Se, or $N-L^1-(Ar^1)_e$;

each $R^d$ independently represents hydrogen, deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups; each $L^1$ independently represents a substituted or unsubstituted aromatic hydrocarbon group having 6 to 30 carbon atoms or a substituted or unsubstituted aromatic heterocyclic group having 3 to 30 carbon atoms, and each $Ar^1$ independently represents a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 these aromatic rings; $L^1$ optionally forms one bond with an aromatic ring adjacent to a heterocycle to which $L^1$ is bonded, directly or via -O-, - S-, $-Si(R^c)_2-$, $-C(R^c)_2-$, or $-NR^c-$, to form a fused ring; each $R^c$ independently represents hydrogen, deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups; and a, b, c, and d each independently represent 0 or 1, and each e independently represents an integer of 0 to 5, provided that, when $R^1$ or $L^1$ does not form a fused ring with an adjacent aromatic ring, $a + b + c + d = 1$ is not satisfied;

[C2]

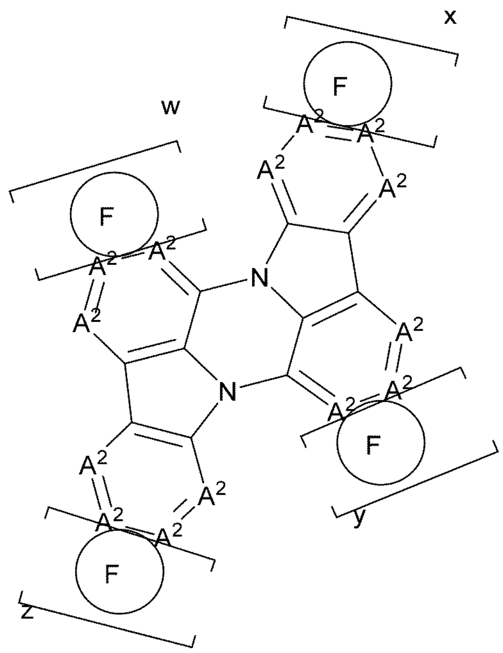

(1－1)

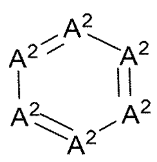

(1－1a)

wherein each $A^2$ independently represents $CR^2$, C or N, provided that the number of N present in one 6-membered ring containing $A^2$ in the general formula (1-1) is 2 or less; each $R^2$ independently represents hydrogen, a cyano group, deuterium, a substituted or unsubstituted diarylamino group having 12 to 44 carbon atoms, a substituted or unsubstituted arylheteroarylamino group having 12 to 44 carbon atoms, a substituted or unsubstituted diheteroarylamino group having 12 to 44 carbon atoms, an aliphatic hydrocarbon group having 1 to 10 carbon atoms, a substituted or unsubstituted aromatic hydrocarbon group having 6 to 30 carbon atoms,

a substituted or unsubstituted aromatic heterocyclic group having 3 to 30 carbon atoms, or a substituted or unsubstituted linked aromatic group formed by linking 2 to 9 such aromatic groups; when $R^2$ represents a substituted or unsubstituted diarylamino group, arylheteroarylamino group, diheteroarylamino group or aromatic hydrocarbon group, or a substituted or unsubstituted aromatic heterocyclic group, $R^2$ optionally forms one or more bonds with an aromatic ring having $A^2$ to which $R^2$ is bonded in the formula (1-1), directly or via -O-, -S-, $-Si(R^b)_2-$, $-C(R^b)_2-$, or $-NR^b-$, to form a fused ring; each $R^b$ independently represents hydrogen, deuterium, an aliphatic hydrocarbon group having 1 to 10 carbon atoms, a substituted or unsubstituted aromatic hydrocarbon group having 6 to 30 carbon atoms, a substituted or unsubstituted aromatic heterocyclic group having 3 to 30 carbon atoms, or a substituted or unsubstituted linked aromatic group formed by linking 2 to 9 such aromatic groups; each ring F independently represents an aromatic ring represented by formula (1-1a), and each ring F is fused with an adjacent ring at any position; and x, y, w, and z each independently represent 0 or 1.

**2.** The emission material according to claim 1, wherein $a + b + c + d \geq 2$ is satisfied.

**3.** The emission material according to claim 2, wherein b and c, or a and d are each 1.

**4.** The emission material according to claim 1, wherein the general formula (1) is represented by the following general formula (2):

[C3]

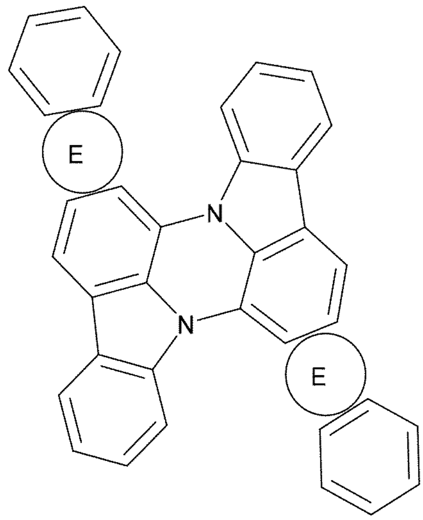

(2)

wherein a ring E has the same meaning as in the general formula (1).

**5.** The emission material according to claim 1, wherein the general formula (1) is represented by the following general formula (3):

[C4]

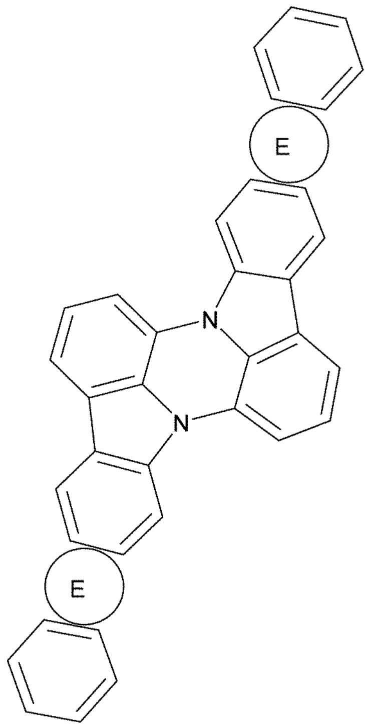

(3)

wherein a ring E has the same meaning as in the general formula (1).

6. The emission material according to claim 1, wherein each ring E in the general formula (1) is independently a group in which $X^1$ in the formula (1a) is represented by $N\text{-}L^1\text{-}(Ar^1)_e$.

7. The emission material according to claim 1, wherein the general formula (1) or the general formula (1-1) is represented by the following general formula (4):

[C5]

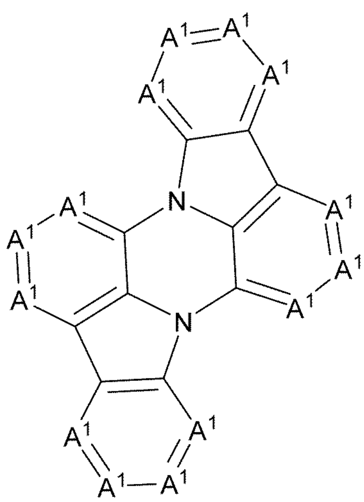

(4)

wherein each $A^1$ independently represents $CR^1$ or N; and $R^1$ has the same meaning as $R^1$ in the general formula (1), provided that the number of N present in one 6-membered ring containing $A^1$ in the general formula (4) is 2 or less.

**8.** The emission material according to claim 1, wherein a difference (ΔEST) between a singlet excited energy (S1) and a triplet excited energy (T1) is 0.40 eV or less.

**9.** The emission material according to claim 1, represented by the general formula (1), wherein at least one $R^1$ or $L^1$ forms one or more bonds with an aromatic ring having $A^1$ to which $R^1$ is bonded or with an aromatic ring adjacent to a heterocycle to which $L^1$ is bonded in the formula (1), directly or via -O-, -S-, $-Si(R^a)_2-$, - $C(R^a)_2-$, or $-NR^a-$, to form a fused ring.

**10.** The emission material according to claim 9, represented by the general formula (1), wherein at least one $L^1$ is directly bonded to an aromatic ring adjacent to a heterocycle to which $L^1$ is bonded, to form a fused ring.

**11.** The emission material according to claim 9, represented by the general formula (1), wherein at least one $R^1$ represents a substituted or unsubstituted carbazolyl group, the carbazolyl group forms one or more bonds with an aromatic ring having $A^1$ to which $R^1$ is bonded in the formula (1), directly or via -O-, -S-, - $Si(R^a)_2-$, $-C(R^a)_2-$, or $-NR^a-$, to form a fused ring.

**12.** The emission material according to claim 1, represented by the general formula (1-1), wherein $x + y + w + z \geq 2$ is satisfied.

**13.** An organic electroluminescent device comprising one or more light emitting layers between an anode and a cathode opposite to each other, wherein at least one of the light emitting layers contains the emission material according to any of claims 1 to 12.

[FIG. 1]

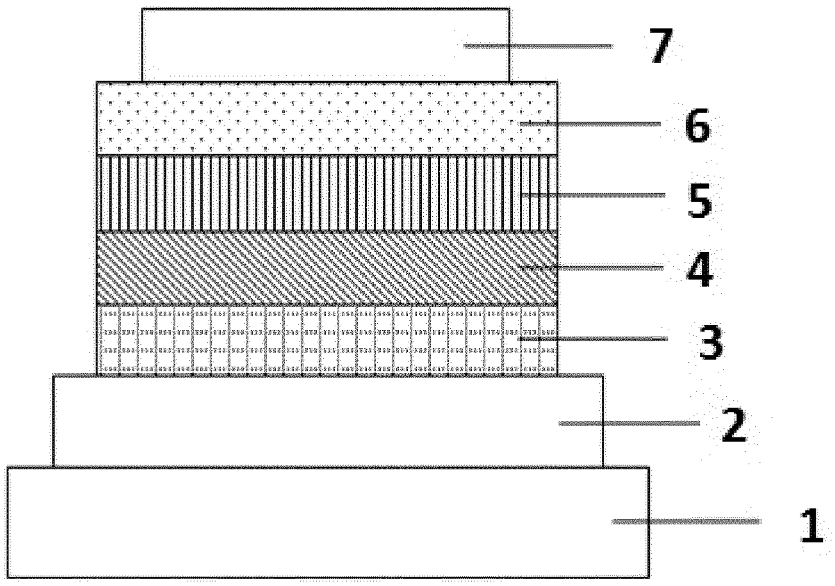

## INTERNATIONAL SEARCH REPORT

International application No.
**PCT/JP2022/015058**

**A. CLASSIFICATION OF SUBJECT MATTER**

***H01L 51/50***(2006.01)i; ***C07D 487/16***(2006.01)i; ***C09K 11/06***(2006.01)i
FI: H05B33/14 B; C09K11/06 650; C07D487/16

According to International Patent Classification (IPC) or to both national classification and IPC

**B. FIELDS SEARCHED**

Minimum documentation searched (classification system followed by classification symbols)

H01L51/50; C07D487/16; C09K11/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | US 2021/0098715 A1 (SAMSUNG DISPLAY CO., LTD.) 01 April 2021 (2021-04-01) entire text, all drawings | 1-13 |
| A | CN 110759925 A (GUANGZHOU CHINA-RAY OPTOELECTRONIC MATERIALS CO., LTD.) 07 February 2020 (2020-02-07) entire text | 1-13 |
| A | KR 10-2018-0008283 A (LG CHEM, LTD.) 24 January 2018 (2018-01-24) entire text, all drawings | 1-13 |
| P, A | KR 10-2021-0096958 A (DUK SAN NEOLUX CO., LTD.) 06 August 2021 (2021-08-06) entire text | 1-13 |

☐ Further documents are listed in the continuation of Box C.
☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 May 2022** | **17 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/015058**

| Patent document cited in search report | Publication date (day/month/year) | Patent family member(s) | Publication date (day/month/year) |
|---|---|---|---|
| US 2021/0098715 A1 | 01 April 2021 | JP 2021-57582 A<br>entire text, all drawings<br>KR 10-2021-0038789 A<br>CN 112582558 A | |
| CN 110759925 A | 07 February 2020 | (Family: none) | |
| KR 10-2018-0008283 A | 24 January 2018 | CN 107619410 A | |
| KR 10-2021-0096958 A | 06 August 2021 | WO 2021/153931 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010134350 A
- WO 2011070963 A
- JP 2012234873 A
- WO 2014008967 A
- CN 111892607

### Non-patent literature cited in the description

- *Journal of Material Chemistry C,* 2017, vol. 5 (3), 709